(19) Europäisches Patentamt

European Patent Office

Office européen des brevets

(11) **EP 1 145 004 B1**

(12) **EUROPEAN PATENT SPECIFICATION**

(45) Date of publication and mention
of the grant of the patent:
**07.04.2004 Bulletin 2004/15**

(51) Int Cl.7: **G01N 33/50**, G01N 33/542,
G01N 33/92, G01N 33/68,
G01N 33/58, C12Q 1/68

(21) Application number: **00900695.8**

(22) Date of filing: **19.01.2000**

(86) International application number:
**PCT/GB2000/000118**

(87) International publication number:
**WO 2000/043780 (27.07.2000 Gazette 2000/30)**

(54) **METHODS FOR DETECTING CHANGES TO CELLULAR COMPONENTS**

METHODE ZUM NACHWEIS VON VERÄNDERUNGEN ZELLULÄRER KOMPONENTEN

DETECTION DE MODIFICATIONS AFFECTANT UN COMPOSANT D'UNE CELLULE

(84) Designated Contracting States:
**AT BE CH CY DE DK ES FI FR GB GR IE IT LI LU
MC NL PT SE**

(30) Priority: **19.01.1999 GB 9901072**

(43) Date of publication of application:
**17.10.2001 Bulletin 2001/42**

(73) Proprietor: **CANCER RESEARCH TECHNOLOGY
LIMITED**
**London WC2A 3NL (GB)**

(72) Inventors:
• **BASTIAENS, Philippe Igor Henri**
**Camden London NW1 0TA (GB)**
• **PARKER, Peter Joseph Jacques**
**Ashtead Surrey KT21 2LG (GB)**

(74) Representative: **Miles, John Stephen**
**Eric Potter Clarkson,**
**Park View House,**
**58 The Ropewalk**
**Nottingham NG1 5DD (GB)**

(56) References cited:
WO-A-97/46714          WO-A-98/45704

• BASTIAENS, P. I. H. ET AL.:
"Microspectroscopic imaging tracks the
intracellular processing of a signal transduction
protein: Fluorescent-labeled protein kinase C
betaI" PNAS, vol. 93, August 1996 (1996-08),
pages 8407-8412, XP000901405 cited in the
application
• BASTIAENS, P.I.H. ET AL.: "CELL BIOLOGY; A
LABORATORY HANDBOOK, 2ND EDITION:
Fluorescence Resonance Energy Transfer
Microscopy; pages 136-146" 1998 , ACADEMIC
PRESS, INC. XP000908803 cited in the
application the whole document
• OSBORNE, S.L. ET AL.: "Calcium-dependent
Oligomerization of Synaptotagmins I and II"
JOURNAL OF BIOLOGICAL CHEMISTRY, vol.
274, 1 January 1999 (1999-01-01), pages 59-66,
XP000906800
• WOUTERS, F.S. ET AL.: "FRET microscopy
demonstrates molecular association of
non-specific lipid transfer protein (nsL-TP) with
fatty acid oxidation enzymes in peroxisomes"
THE EMBO JOURNAL, vol. 17, no. 24, 1998,
pages 7179-7189, XP000891741

**Description**

The present invention relates to methods for detecting changes to a macromolecular component of a cell; in particular, the method relates to the use of fluorescent molecules for detection and the use of fluorescence resonance energy transfer (FRET) methods, and more particularly it relates to fluorescence lifetime imaging.

Fluorescent assays for biological systems have been used for several years since, compared to many biological techniques, they have the advantage of, in some circumstances, being able to be carried out non-invasively and also they are able to give real time analysis of particular reactions in complex biological systems in which many reactions are being carried out simultaneously. In conjunction with the development of physical techniques for fluorescent assays has been the development of biological reporter constructs which serve as monitors of reactions, for example, within a cell. In particular, the development of fluorescent proteins that do not require cofactors for their intrinsic fluorescence has meant that such proteins can be introduced into and expressed in cells *via* genetic constructs.

Examples of biological reporters for fluorescent assays, and details of intrinsically fluorescent proteins, notably so-called "green fluorescent proteins" or "GFPs" (although they may emit blue or yellow light) are known. Miyawaki *et al* (1997) *Nature* **388,** 882-887 describes a GFP-based $Ca^{2+}$ sensing system; Mitra *et al* (1996) *Gene* **173,** 13-17 describes a two-GFP-based system for use in identifying protease inhibitors; WO 97/28261 discloses a two-GFP system in which the GFP donor and GFP acceptor are linked by a peptide containing a protease cleavage site. WO 95/07463 describes uses of GFPs; WO 96/23898 relates to a method of detecting biologically active substances using GFPs; Heim & Tsien (1996) *Current Biology* **6,** 178-182 relates to engineered GFPs with improved brightness, longer wavelengths and fluorescence resonance energy transfer (FRET); Poppenborg *et al* (1997) *J. Biotechnol*. **58,** 79-88 relates to GFPs as a reporter for bioprocess monitoring; Park & Raines (1997) *Protein Science* **6,** 2344-2349 relates to a GFP as a signal for protein-protein interactions; Niswender *et al* (1995) *J. Microscopy* **180,** 109-116 relates to quantitative imaging of GFP in cultured cells; Chalfie *et al* (1994) *Science* **263,** 802-805 relates to GFP as a marker for gene expression; Hampton *et al* (1996) *Proc. Natl. Acad. Sci. USA* **93,** 828-833 relates to the *in vivo* examination of membrane protein localisation and degradation with GFP; Heim *et al* (1995) *Nature* **373,** 663-664 relates to mutant GFPs with altered fluorescent properties; Mosser *et al* (1997) *BioTechniques* **22,** 150-161 relates to the use of a dicistronic expression cassette encoding GFP for the screening and selection of cells expressing inducible gene products; Suarez *et al* (1997) *Gene* **196,** 69-74 relates to GFP-based reporter systems for genetic analysis of bacteria; Niedenthal *et al* (1996) *Yeast* **12,** 773-778 relates to GFP as a marker for gene expression and subcellular localisation in budding yeast; Mahajan *et al* (1998) *Nature Biotech.* **16,** 547-552 relates to the probing of Bcl-2 and Bax interactions in mitochondria using GFPs and FRET; and Prescott *et al* (1997) *FEBS Lett* **411,** 97-101 relates to the use of GFP as a marker for assembled mitochondrial ATP synthase in yeast. GFPs and their uses have been reviewed in Pozzan *et al* (1997) *Nature* **388,** 8340-835, Misteli & Spector (1997) *Nature Biotechnology* **15,** 961-964; and Cubitt *et al* (1995) *Trends Biochem. Sci.* **20,** 448-455. Reporter systems which are not based on GFPs but which may find use in biological systems are known, for example in Zlokarnik *et al* (1998) *Science* **279** 84-88; Bastiaens & Jovin (1996) *Proc. Natl. Acad. Sci. USA* **93,** 8407-8412; and Bastiaens *et al* (1996) *EMBO J.* **15,** 4246-4253. Szollosi *et al* (1998) *Cytometry* **34,** 159-179 is a review of the application of FRET technology in the clinical laboratory.

WO 97/08538 relates to fluorescence lifetime-based imaging and spectroscopy in tissues.

WO 94/18547 relates to apparatus for quantitative imaging of multiple fluorophores.

WO 92/13265 relates to method and apparatus for multi-dimensional phase fluorescent lifetime imaging.

WO 92/07245 relates to method and apparatus for performing phase fluorescence lifetime measurements in flow cytometry.

Our co-pending PCT Patent Application No PCT/GB99/02597 relates to methods of multi-frequency fluorescence lifetime imaging (mfFLIM).

Our co-pending PCT Patent Application No PCT/GB99/02598 relates to methods for the measurement of FRET taking place between a donor and an acceptor system, and to biological constructs for detecting a change in the degree of FRET taking place between donor and acceptor chromophores.

Our co-pending PCT Patent Application No PCT/GB99/02596 relates to novel GFPs.

There remains the need for further systems for detecting the molecular state, or changes in. the molecular state of macromolecules, particularly those that are present in a living cell and which state, or changes in the state, can be detected in the living cell. Similarly, there remains the need for further systems for detecting the molecular state of macromolecules in historic samples, particularly samples that have been derived from or are associated with pathological tissue.

A first aspect of the invention provides a method of detecting the state, or the change in state, of a macromolecular component of a cell which can exist in a first state or a second state which differ by virtue of a covalent modification or which are different conformations wherein in its first state the macromolecular component is substantially incapable of being bound by a sensor molecule and in its second state the macromolecular component is able to be bound by the sensor molecule, the method comprising the steps of (1) providing a sample comprising the macromolecular component wherein the macromolecular component is bound to a donor fluorophore; (2) contacting the sample provided in (1) with a sensor molecule which is able to bind the macromolecular component in its second state but is substantially incapable of binding the macromolecular component in its first state wherein the sensor molecule is bound to an acceptor molecule; and (3) detecting fluorescence resonance energy transfer (FRET) between the donor fluorophore bound to the macromolecular component and the acceptor molecule bound to the sensor molecule wherein FRET is measured by the decrease in the fluorescence lifetime of the donor fluorophore.

The acceptor molecule is preferably a chromophore; the acceptor chromophore need not be (but may be) a fluorescent molecule but the donor is.

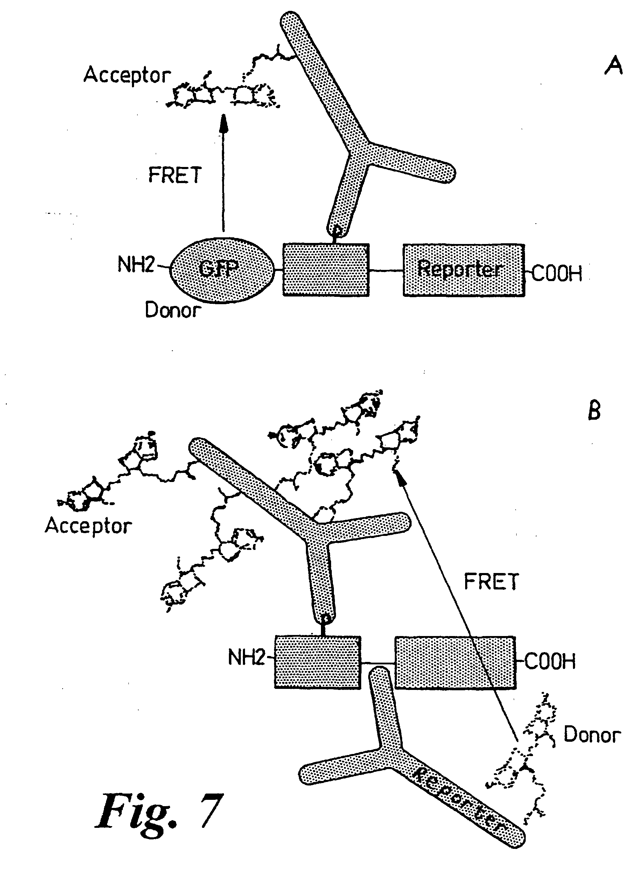

When the sensor molecule binds to the macromolecular component in its second state, the donor and acceptor molecules are sufficiently close in proximity for FRET to occur. It will be appreciated that, as described in more detail below, the donor fluorophore is bound closely to the macromolecular component and that, similarly, the acceptor molecule is bound closely to the sensor molecule such that the occurrence of FRET between the donor and acceptor molecules is indicative of an interaction occurring between the sensor molecule and the macromolecular component when in the correct state to be bound (ie that the sensor molecule is binding selectively to the macromolecular component). Thus, the altered state of the macromolecule in question is detected by fluorescence resonance energy transfer (FRET) between the donor fluorophore bound to the macromolecular component in question and the acceptor on the sensor (an example of this is depicted in Figure 7).

The second state of the macromolecular component to which the sensor binds exists in the absence of the sensor molecule. Typically, and preferably, the first and second states of the macromolecular component are states in which the macromolecular component exists in the cell.

Typically, the decrease in the fluorescence lifetime of the donor fluorophore is measured by fluorescence lifetime imaging; it is particularly preferred if the fluorescence lifetime of the donor fluorophore is measured by fluorescence lifetime imaging microscopy (FLIM). Suitable methods of measuring fluorescence lifetime of the donor fluorophore, and methods of FLIM are well known in the art, some of which are described in more detail in the Examples. For example, suitable methods are described in Bastiaens & Jovin (1996) *Proc. Natl. Acad. Sci. USA* **93,** 8407-8412; Gadella *et al* (1993) *Biochem. Chem.* **48,** 221-239; Gadella *et al* (1994) *Bioimaging* **2,** 139-159; Gadella *et al* (1993) *Biophys. J.* **64,** A130; Carlsson & Liljeborg (1997) *J. Microsc.* **185,** 37-46; Dong *et al* (1995) *Biophys. J.* **69,** 2234-2242; Lakowicz & Berndt (1997) *Rev. Sci, Instrum.* **62,** 1727-1734; Sanders *et al* (1995) *Anal. Biochem.* **227,** 302-308; Schneider & Clegg (1997) *Rev. Sci. Instrum.* **68,** 4107-4119; So *et al* (1995) *Bioimaging* **3,** 1-15; and Squire & Bastiaens (1999) *J. Microsc.* **193,** 36-49.

It is particularly preferred if the fluorescence lifetime measurement is spatially resolved since this gives additional information about where within the sample (for example, where within the cell) the different states of the macromolecular component occur. Conveniently, spatial resolution may be obtained using suitable fluorescence microscopic methods which are well known in the art, some of which are described in the Examples. Thus, when the method is carried out on live cells in order to determine the state or change in state of a macromolecular component, for example under certain biological conditions such as in the presence or absence of a growth factor or hormone or the like, it is preferred if the decrease in the donor lifetime is measured by FLIM so that information about where within the cell (for example, in the nucleus, cytosol, associated with a particular membrane) the particular state of the macromolecular component is, or where within the cell there is a change in the state of the macromolecular component following, for example, administration of a growth factor or hormone. However, spatial resolution can be carried out other than by microscopic methods. Spatial resolution is also particularly preferred when the methods of the invention are used in drug screening and the like as is described in more detail below.

In a preferred embodiment, fluorescence lifetimes of the donor molecules may, for example, be determined in cells and tissues by the following steps:

Irradiation of donor fluorophore bound to the macromolecular component with a beam of intensity modulated excitation energy with a wavelength which excites preferentially the donor on the macromolecular component.

Receiving fluorescence emission from only the donor fluorophore by using narrow band pass filter which blocks out the acceptor fluorescence on the sensor.

Determination of a phase lifetime ($\tau_p$) and modulation lifetime ($\tau_M$) from the phase shift and demodulation of the fluorescence emission relative to the excitation light.

When the donor fluorophore exhibits multi-exponential decay multi-frequency fluorescence lifetime imaging microscopy (mfFLIM) can be applied to obtain all the decay components with their characteristic lifetimes. The method of mfFLIM is described in our co-pending PCT Patent Application No PCT/GB99/02597 entitled Multiple Frequency Fluorescence Lifetime Imaging. An advantage of using this method compared to using other FLIM methods is that it is possible to quantify the populations of the macromolecular states in each spatially resolvable element without knowing the lifetimes of the donor fluorophore in the absence of, and presence of 100 % of, bound sensor.

The populations of proteins that are in a specific macromolecular state at each spatially resolvable element of an image can be estimated by exploiting prior knowledge about the macromolecular system under study in a global analysis (Beechem, J.M. (1992) *Meth*. *Enzym*. **210**, 37-54) of the mfFLIM data (Verveer *et al* (2000) *J. Microsc*., in press). A typical application is a protein interaction system where it can be assumed that only two states are present: bound and unbound. Tagging of this binary protein system with a suitable donor/acceptor chromophore pair results in efficient FRET only in the bound state reducing the donor fluorescence lifetime for that state to a discrete value. Mixing of the bound/unbound protein states results in double exponential fluorescence decays for the donor at each pixel with spatially invariant lifetimes ($\tau_f$, $\tau$; global parameters) and spatially varying amplitudes ($\alpha$; local parameters). The assumption of distinct discrete lifetimes is valid since it is likely that the spatial configuration of the donor and acceptor dyes is consistent for each protein complex. The global analysis is implemented by simultaneously analyzing all pixels in the image subject to the constraint that the lifetime values of each molecular species are equal in all pixels. The results of the calculation are the two spatially invariant lifetime values and an image of the bound/unbound (reacted/unreacted) protein populations. This implies that the reference donor lifetime measurement in absence of acceptor is not necessary, a distinct advantage in live cell imaging. This analysis can be simultaneously performed on several independent mfFLIM data sets as long as the assumption holds that the lifetime values of each molecular species are invariant. The result of this approach is a dramatic improvement in accuracy of the estimated lifetimes and the corresponding fractional populations of each fluorescent species. A further advantage of this type of analysis is the possibility to estimate populations of two molecular species with single frequency FLIM without prior knowledge of the two associated lifetime. The true lifetimes of the donor in the presence and absence of the acceptor are also obtained in a single analysis allowing the calculation of the true FRET efficiency in the protein complex.

Due to transfer of energy *via* dipole-dipole coupling to the acceptor moiety, the average time the donor spends in the excited state will decrease as detected by its average excited state lifetime. Typically, the fluorescence lifetime of the fluorophore bound to the macromolecular component is measured at each pixel in the cell in the absence of the sensor. This reference donor lifetime measurement in the absence of acceptor can be made on the same specimen by photobleaching the acceptor dye by excitation in its absorption maximum (Wouters & Bastiaens (1999) *Curr. Biol*. **9,** 1127-1130). The average lifetime ($\bar{\tau}$) over the whole image is then calculated and used to calculate the FRET efficiency ($E_i$) at each pixel (i) by measuring the lifetime ($\tau_i$) of the reporter at each pixel i in the cell in the presence of the sensor via the following relation:

$$E_i = 1 - \tau_i / \bar{\tau}$$

The occurrence of FRET on the molecular level is discretely dependent upon the protein state due to the acute $R^{-6}$ distance dependence of the FRET efficiency. In other words, the fluorescent molecules are either in close proximity exhibiting efficient FRET or too far apart for detectable FRET. Consequently, on the microscopic scale, the measured FRET efficiency at each pixel in an image is a linear function of the population of protein states. These populations can thus be approximately calculated from FRET efficiency images of a two component interacting protein system when the true FRET efficiency in the protein complex is known.

When the FRET efficiency is known under conditions of 100% molecular transition or modification ($E_f$), the extent of the reaction as a percentage ($Q_i$) can be calculated at each pixel i by the following relation:

$$Q_i = (E_i / E_f) \times 100$$

When global analysis is used on mfFLIM data, the extend of the reaction $Q_i$ equals the calculated population images $\alpha_i$ of reacted species at pixel i. The FRET efficiency at 100% molecular transition or modification ($E_f$) is calculated from the two spatial invariant lifetimes obtained from this analysis:

$$E_f = 1 - \tau_f / \tau$$

where $\tau_f$ and $\tau$ are the spatial invariant lifetimes of the fully reacted and unreacted species respectively.

FRET measurements *via* donor photophysical properties by using for example FLIM, has a specific advantage over steady-state spectral ratio methods since FRET is detected through the excited state properties (the fluorescence lifetime) of the donor only. As a consequence, the acceptor emission does not affect the FRET efficiency calculation and therefore the acceptor concentration does not need to be determined or controlled in the experiment. This also implies that if one uses an acceptor-labelled sensor which binds to a donor labelled reporter, there is no requirement for absolute specificity of the sensor for the reporter. Only the sensors which are in close molecular proximity of the donor labelled reporter are detected through FRET *via* a decrease in lifetime of the donor on the reporter, whereas all the non binding sensors are "invisible" to the experiment. This has the practical advantage that the concentration of the sensor molecule does not have to be controlled so long as it is in excess. This is particularly useful when conducting experiments in which very small volumes of samples are used, such as in introducing samples of volume between 10 and 100 fl into a living cell or in using very small volumes of samples in high throughput drug screening programmes. It is also useful in situations, for example in high throughput drug screens, where the concentration of various components in the screen, such as the sensor as described in more detail below, may be difficult to control.

There are two general methods for the determination of fluorescence lifetimes. In the time domain:(a), the fluorescence lifetime is directly measured upon excitation of the sample with a short pulse of light, where the duration of the pulse is ideally much shorter than the fluorescence lifetime ($\tau_\rho$) to be measured. This results in a fluorescence emission whose intensity decreases exponentially with time. For a sample comprising a single fluorescent species, the fluorescence lifetime is given by the time over which the fluorescence intensity drops to about 37 % of its initial value; (b) In the frequency domain, the sample is excited with sinusoidally modulated light, the optimal angular frequency of which is reciprocal to the fluorescence lifetime to be measured. This results in the re-emission of sinusoidally modulated fluorescence at the same frequency ($\omega$) as the excitation but with a reduction in the relative modulation depth, $M(\omega)$, and shifted in phase, $\Delta\Phi(\omega)$. The phase shift and relative modulation depth can be used to calculate the phase $\tau_\Phi(\omega)$ and modulation $\tau_M(\omega)$ lifetimes, respectively. The phase and modulation lifetimes are equal only in samples comprising a single fluorescent species. For heterogeneous samples comprising more than one fluorescent species or where a fluorescent species exhibits more than one exponential component, the phase and modulation lifetimes will differ, in this situation, the true lifetime composition can be obtained from measurements of the phase-shift $\Delta\Phi(\omega)$ and de-modulation -$M(\omega)$ over a range of frequencies and fitting the results to a set of dispersion relationships (Gratton, E. and Limkeman, M. (1983) *Biophys. J.* **44,** 315-324; Lakowicz, J.R. and Maliwal, B.P. (1985) *Biophys. Chem.* **21,** 61-78).

Donor photobleaching may be used to measure the decrease in donor fluorescence lifetime, but this method leads to destruction of the fluorophore and so is irreversible. Thus, measurement using fluorescence lifetime imaging is preferred. Data acquisition in fluorescence lifetime imaging is, in any case, much more rapid than donor photobleaching, being in the order of $10^{-1}$ to 10 seconds.

The macromolecular component of the cell whose state, or change in state, is detected may be any macromolecular component which exists in two states and that at least for one of the states a sensor molecule may be made which binds to the macromolecular component in that state but does not substantially bind to the macromolecular component in the other state. Typically, the macromolecular component of a cell may be any of a protein, a RNA molecule, a DNA molecule or a lipid.

Proteins, particularly enzymes, are well known to exist in different states within a cell and the changes in the state of proteins, particularly enzymes, occur during many life processes such as cell signalling processes. Typically, a protein molecule is translated in a cell and exists in a first state and it may be post-translationally modified into a second state. For example, in a first state the protein may not be phosphorylated, or acylated, or methylated, or sulphated at any given amino acid residue, or have ubiquitin or ubiquitin-like proteins, or a farnesyl group, or sugar residues (glycosylation) or fatty acid residues (such as myristyl groups) attached whereas in a second state the protein may be phosphorylated, or acylated, or methylated, or sulphated at any one or more given residues, or have ubiquitin or ubiquitin-like like proteins, or a farnesyl group, or sugar residues or fatty acid residues (such as myristyl groups) attached. Sensor molecules which recognise and bind to, for example, the protein phosphorylated at a given site are available, such as antibodies which recognise and bind to phosphoserine, or phosphothreonine, or phosphotyrosine residues. As has been described above, it is not necessary for the sensor molecule to be specific for the macromolecular component provided that it does bind the macromolecular component in its second state (whether or not it can bind other components of the cell) and it is substantially incapable of binding the macromolecular component in its first state. By way of example, antibodies which bind phosphotyrosine, or phosphoserine, or phosphothreonine are useful in detecting changes in the phosphorylation state of a particular macromolecular component even though they are able to bind to these phosphorylated residues on proteins other than the particular macromolecular component. Similarly, antibodies which are able to bind ubiquitin (or other ubiquitin-like proteins) are useful in detecting ubiquitinylation of the macromolecular component of interest (in this case a protein) even though the anti-ubiquitin antibodies are able to bind other ubiquitinylated proteins and free ubiquitin. It will be appreciated that the specificity of the system is conferred by the donor fluorophore bound to the macromolecular component in question. Nevertheless, it is preferred if the sensor molecule is also selective for, or specific for, the particular macromolecular component.

It will be appreciated that more than one donor fluorophore may be linked to the macromolecular component.

It is particularly preferred, but not essential, that the second state of the macromolecule is different to the first state of the macromolecule by virtue of a covalent modification. Sensors which are able to detect covalent modifications to a macromolecular component (ie where in the first state the component is unmodified and in the second state the component is covalently modified) include antibodies which bind selectively to particular phosphorylated proteins (for example, the antibody described in the Examples which binds PKC$\alpha$ when it is phosphorylated on threonine 250 but not when threonine 250 is not phosphorylated; an antibody which is specific for phosphorylation of Thr412 of p70S6 kinase (Weng *et al* (1998) *J Biol Chem* **273,** 16621-16629); monoclonal antibodies which are specific to tyrosine phosphorylation sites in the acetylcholine receptor (Tzartos *et al* (1995) *FEBS Lett* **363,** 195-198)); antibodies which will detect individual acetylation sites in histone H4 (Turner *et al* (1989) *FEBS Lett* **253,** 141-145); an antibody which will recognise ε-N-acetyl-lysine (ie any protein acetylation site; Hebbes *et al* (1989) *Mol Immunol* **26,** 865-873); antibodies specific for galactosylation (Coon & Weinstein (1983) **11,** 173-205); and monoclonal antibodies that specifically recognise different sulphated forms of chondroitin (Mark *et al* (1990) *Differentiation* **43,** 37-50).

It will be appreciated that all of these selective binding molecules may be useful as sensor molecules, and that in any case suitable sensor molecules, especially antibodies, may be made which detect covalently modified proteins, but not unmodified said proteins, using methods well known in the art, some of which are described below.

Proteins are also known to exist in different conformations, that is to say that the proteins have the same primary structure, but they are folded differently in three dimensions. In one such conformation (or state) the protein may not have an epitope which is capable of being bound by a particular antibody, whereas in a second conformation (or state) an epitope is present which is accessible to, and may be bound by, the particular antibody. Proteins which are known to exist in different conformational states include p53, prion proteins, protein kinase C and $\beta_2$-integrin. Monoclonal antibodies which are sensitive to the conformation of p53 are described in Yewdell *et al* (1986) *J Virol* **59,** 444-452; Receptor of Activated Protein Kinase C (RACK1) binds to activated conformations of protein kinase C$\alpha,\beta,\gamma$ (Ron *et al* (1994)*Proc Natl Acad Sci USA* **91,** 839-843); and a monoclonal antibody which exhibits a conformationally-dependent interaction with $\beta_2$-integrin is described in McDowall *et al* (1998) *J. Biol. Chem.* **273,** 27396-27403.

The sensor molecule may be any suitable sensor molecule which is able to bind the macromolecular component in its second state but is substantially incapable of binding the macromolecular component in its first state. It will be appreciated that a convenient sensor molecule is an antibody. Antibodies may be prepared which, for example, bind to phosphoserine, phosphothreonine and phosphotyrosine residues in proteins but do not substantially bind to the equivalent non-phosphorylated residues.

Antibodies which bind to ubiquitin may be obtained from Sigma-Aldrich Company Ltd, Fancy Road, Poole, Dorset BH12 4QH, UK under product number U5379.

By "antibody" we mean whole antibodies or fragments of antibodies which retain antibody-like binding characteristics or variants or derivatives of antibodies which retain antibody-like binding characteristics. In particular, the term antibodies specifically includes Fab-like molecules (Better *et al* (1988) *Science* **240,** 1041); Fv molecules (Skerra *et al* (1988) *Science* **240,** 1038); single-chain Fv molecules where the $V_H$ and $V_L$ chains are linked by a flexible oligopeptide (Bird *et al* (1988) *Science* **242,** 423; Huston *et al* (1988) *Proc Natl Acad Sci USA* **85,** 5879); and single domain antibodies (dAbs) comprising isolated V domains (Ward *et al* (1989) *Nature* **341,** 544).

It will be appreciated that with today's technology, suitable sensor molecules to detect one state of a macromolecular component and not another state of the macromolecular component may be made. For example, single chain Fv molecules may be cloned for display of associated heavy and light chain variable domains on the surface of filamentous bacteriophage (Winter *et al* (1994) *Annu. Rev. Immunol*. 12, 433-455). This allows screening of the phage display for single chain Fv molecules which are useful as sensors provided that suitable selection systems are available. Suitable selection systems include phosphorylated/non-phosphorylated peptide pairs, where the phosphorylation site represents the phosphorylation site in the macromolecular component (protein) of interest. Thus, such phage display libraries are screened with phosphopeptides representing consensus sequences for specific protein kinases (a list of such consensus sequences is described in Kemp and Pearson (1990) *TIBS* 15, 342-346). Phages identified in such screens should harbour the DNA encoding single chain Fvs recognising the phosphorylated consensus sequence peptide, and these are screened to determine whether they also bind the non-phosphorylated peptide. Those that bind phosphorylated, but not non-phosphorylated, are believed to be suitable sensors.

Particularly when the macromolecular component is a protein, and in its second state the protein is phosphorylated, a number of non-antibody sensors are available. For example, the insulin receptor substrate IRS-1 (Kahn *et al* (1993) *Recent Prog*. *Horm*. *Res*. 48, 291-339) and SHG protein (Pelicci *et al* (1992) *Cell* 70, 93-104) are two proteins with a phosphotyrosine binding domain (PTB) *via* which they bind to receptor tyrosine kinases with the consensus sequence NPX-p Y. (Pawson (1995) *Nature* **373,** 573-580). Interaction depends completely on tyrosine phosphorylation. Thus a suitable sensor of receptor tyrosine phosphorylation is, for example, the PTB domain of SHC (amino acids 1-238 of SHC) or of IRS-1

As another example, Pin1 is a conserved mitotic peptidyl-prolyl isomerase. In response to their phosphorylation on serine or threonine residues during mitosis, Pin1 binds and regulates a set of proteins with a W-F-Y-pS/pT-R-R related consensus sequence (Yaffe *et al* (1997) *Science* 278, 1957-1960; www.sciencemag.org/feature/data/974519.sh1). Pin1 is therefore a suitable sensor molecule for detecting changes in phosphorylation state of these proteins.

In a further method, the second state of the macromolecular component may be brought about by the macromolecular component binding non-covalently to another moiety. Thus, in the first state the macromolecular component is not bound to the further moiety, but in the second state it is so bound. It will be appreciated that the sensor molecule in this situation may be one which binds the further moiety (thus detecting the second state of the macromolecular component in which it is bound to the further moiety) or it may be one which binds to the macromolecular component-further moiety bound complex but which does not bind to either the macromolecular component or further moiety when not complexed together. It will be appreciated that in a typical scenario the macromolecular component is a first protein, the further moiety is a further protein and the sensor molecule is an antibody which binds the further protein but does not bind the first protein. Thus, the method of the invention can be used to determine when the first protein is not bound by the further protein, and when it is so bound.

Lipids, particularly inositol phospholipids, are known to exist in different states within a cell and changes in the state, for example in the state of phosphorylation of a given inositol phospholipid, are associated with, for example, cell signalling. Protein binding domains which can be used as sensors which bind to specific phosphoinositide lipids are known in the art. These include the Pleckstrin Homology (PH) domain which binds $PI4,5P_2$ or $PI3,4,5P_3$ (reviewed in Bottomley *et al* (1998) *Biophys Acta* **1436,** 165-183) and FYVE domains that bind PI3P (Burd & Emr (1998) *Mol. Cell* **2,** 157-162).

Typically, the donor fluorophore is bound to one of the acyl chains of the lipid as is known in the art. Thus, chemically synthesised fluorescent phosphatidylinositol may be employed as the donor. Short acyl chain phosphatidylinositol that can more easily penetrate cells would be the preferred donor. As an alternative, the donor may be attached to the PIP3 or PIP2 molecule using a suitable binding protein (as described above), and the acceptor may be bound to a lipid which may be intercalated in a membrane and which has no or low specificity.

DNA, particularly chromosomal DNA, is known to exist in different states within a cell and changes in the state are, for example, associated with changes in gene expression. Methylation of DNA at particular regions of a chromosome associated with a gene is believed to be involved in the control of gene expression. Thus, in a first state the DNA at or near to the gene may be substantially unmethylated, whereas in the second state the DNA may be methylated at or near to the said gene. The donor fluorophore may be linked to the DNA molecule (ie macromolecular component) in a selective fashion, for example at or near to a particular gene, by using a nucleic acid probe which binds selectively at the or near the gene of interest or other site in the DNA where a change from a first state to a second state is to be detected (eg methylation). Methods of attaching suitable donor or acceptor molecules to a nucleic acid probe are well known in the art, some of which are described below. Suitable sensor molecules include non-cleaving mutants of restriction enzymes which bind to methylated DNA but not to non-methylated DNA, or non-cleaving mutants of restriction enzymes which bind to non-methylated DNA but not methylated DNA.

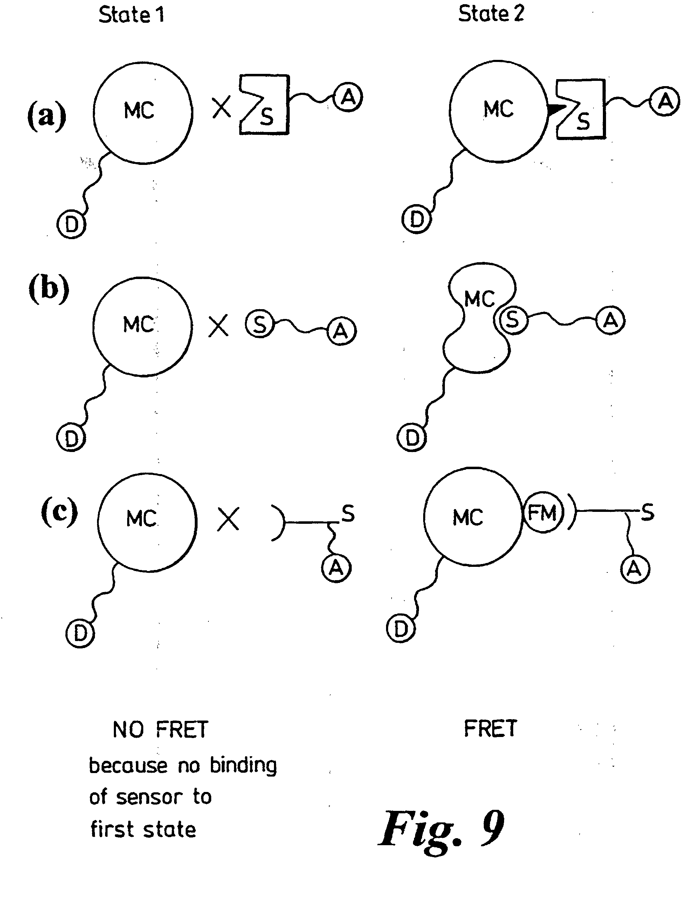

Various examples of the possible scenarios are depicted in Figure 9.

The donor fluorophore may be either directly or indirectly linked to the macromolecular component. By "directly linked" we include that the donor fluorophore and the macromolecular component are covalently linked. By "indirectly linked" we include that the donor fluorophore and the macromolecular component are linked *via* a further moiety. For example, and as is described in one preferred embodiment described below, the donor fluorophore and the macromolecular component are linked *via* an antibody wherein the antibody is able to bind selectively and tightly to the macromolecular component and the donor fluorophore is covalently attached to the antibody. Typically, the antibody binds with a binding constant of between $10^{-7}$ and $10^{-11}$ M. It is preferred if the binding constant is at least $10^{-7}$ M, more preferably at least $10^{-8}$ M, and still more preferably at least $10^{-9}$ M or $10^{-10}$ M. Typically, in this embodiment the fluorophore covalently attached to the antibody is a non-protein fluorophore.

In one embodiment of the invention where the macromolecular component is a protein, the donor fluorophore is a further, fluorescent protein and the protein and the fluorescent protein are fused so that the fused protein may be encoded by a nucleic acid. Preferably, the fluorescent protein is a GFP.

In a further embodiment, the donor fluorophore is indirectly, but selectively and tightly, linked to the macromolecular component by virtue of an intermediate binding moiety wherein the intermediate binding moiety binds directly and selectively and tightly to the macromolecular component and the donor fluorophore binds either directly or indirectly to the intermediate binding moiety. Typically, 90% of all of the intermediate binding moieties should be bound to the macromolecular component. For example, the intermediate binding moiety may be a protein in the cell which is known to bind to the macromolecular component in question in both the first state and the second state. Typically, the donor fluorophore is a GFP and the GFP is fused to the intermediate binding moiety (protein). Also typically, the donor fluorophore is covalently bound to an antibody which binds tightly and selectively to the intermediate moiety.

In a preferred embodiment the donor and acceptor molecules are polypeptides which require no cofactor in order to be fluorescent (ie the polypeptides are intrinsically fluorescent once synthesised). Particularly preferred donors and acceptors include "green fluorescent proteins" (GFPs) which have suitable spectral characteristics in order to behave, as the case may be, as a donor or acceptor in a fluorescence resonance energy transfer (FRET) reaction. GFPs are produced naturally by *Aequorea victoria* but, as is well known in the art and described, for example, in Mitra *et al* (1996) *Gene* **173,** 13-17; Cubitt *et al* (1995) *Trends Biochem. Sci.* **20,** 448-454; Miyawaki *et al* (1997) *Nature* **388,** 882-887; Patterson *et al* (1997) *Biophys. J.* **73,** 2782-2690; Heim & Tsien (1996) *Curr. Biol.* **6,** 178-182; and Heim *et al* (1995) *Nature* **373,** 663-664, mutant GFPs are available which have modified spectral characteristics. Certain GFPs and mutant GFPs are available from Clontech Laboratories UK Ltd, Wade Road, Basingstoke, Hants RG24 8NE. Fluorescent proteins from nonbioluminescent Anthozoa species may also be useful (see Matz *et al* (1999) *Nat. Biotechnol.* **17,** 969-973.

Fluorescence resonance energy transfer (FRET) is the physical process by which energy is transferred from an excited donor to another molecule (the acceptor) by means of long-range dipole-dipole coupling. The spectroscopic requirements for effective transfer over distances of 1-10 nm, which is the relevant distance in the method of the invention are: (1) substantial overlap between the fluorescence spectrum of the donor and the absorbance spectrum of the acceptor defined by the overlap integral, (2) adequate quantum yield of the donor (>0.1) and a sufficiently high absorption coefficient of the acceptor ($\varepsilon_a$ > 10000 M$^{-1}$ cm$^{-1}$) and (3) the transition dipoles of donor and acceptor must be either oriented favourably relative to each other or one or both must have a certain degree of rapid rotational freedom.

Typical donor/acceptor pairs are given in Wu & Brand (1994) Resonance Energy transfer: methods and applications. *Anal Biochem.* **218,** 1-13. Spectroscopic characteristics of indocyanine (Cy3, Cy3.5, Cy5, Cy7) donor/acceptor pairs are given in Bastiaens & Jovin (1998) Fluorescence resonance energy transfer microscopy, *in* Cell biology, a laboratory handbook (Vol 3) (Celis, J. E., ed.), 136-146, Academic Press.

The following are some other useful examples of donor/acceptor pairs, all of which are commercially available from the sources indicated (Clontech Laboratories Inc, 1020 East Meadow Circle, Palo Alto, CA 94303-4230, USA; Molecular Probes Inc, 4849 Pitchford Avenue, Eugene, OR 97402-9165; Amersham International plc, Amersham Place, Little Chalfont, Bucks HP7 9NA, UK). The donors are categorised according to the spectral region of fluorescence emission.

Blue/cyan emitters:    Enhanced blue fluorescent protein (EBFP), Clontech Enhanced cyan fluorescent protein (ECFP), Clontech

Green emitters:    Enhanced green fluorescent protein (EGFP), Clontech Enhanced yellow fluorescent protein (EYFP), Clontech
Fluorescein, Molecular Probes
Oregon Green 488, Molecular Probes
Alexa 488, Molecular Probes
Bodipy FL, Molecular Probes
Cy2, Amersham

Yellow/red emitters:    Rhodamine 6G, Molecular Probes
Alexa 546, Molecular Probes
Alexa 568, Molecular Probes
Bodipy TMR, Molecular Probes
Cy3, Amersham
Cy3.5, Amersham
DsRed, Clontech

Far red emitters:    Cy5, Amersham
Alexa 594, Molecular Probes
Bodipy 581/591, Molecular Probes

Each category of donors can have several possible acceptor dyes. Below are some examples of acceptor molecules for each category of donor fluorophores given above.

| Donor | Acceptor |
| --- | --- |
| Blue/cyan emitters | EGFP, Clontech |
| | EYFP, Clontech |
| | Cy2, Amersham |
| | Fluoroscein, Molecular Probes |
| | Alexa 488, Molecular Probes |
| | Bodipy FL, Molecular Probes |
| Green emitters | Rhodamine 6G, Molecular Probes |
| | Alexa 546, Molecular Probes |

(continued)

| Donor | Acceptor |
|---|---|
| | Alexa 568, Molecular Probes |
| | Bodipy TMR, Molecular Probes |
| | Cy3, Amersham |
| | Cy3.5, Amersham |
| | DsRed, Clontech |
| | Cy5, Amersham |
| | Malachite Green, Molecular Probes |
| Yellow/red emitters | Cy5, Amersham |
| | Cy7, Amersham |
| Far red emitter | Cy7, Amersham |

Malachite green is a good acceptor molecule but is not fluorescent.

In general, non fluorescent molecules that absorb at the emission wavelength of the donor can be used as acceptors with the described method of measuring FRET via donor lifetimes.

The donor or acceptor molecules may be linked to the sensor molecules, or to the macromolecule component, using any suitable method, many of which are well known in the art. In particular, methods of linking suitable molecules to proteins are well known in the art.

There are two main classes of reactive groups by which the donor or acceptor molecules can be conjugated to proteins: thiol-reactive groups and amine-reactive groups. Typical thiol reactive groups include iodoacetamides and maleimides, typical amine-reactive groups include isothiocyanates, sulfonyl chlorides and succinimidyl esters. Details are given in Haugland R.P. "Handbook of Fluorescent Probes and Research Chemicals", Molecular Probes, sixth edition (1996), ISBN 0-9652240-0-7 (Spence, MTZ, ed). Oligonucleotides can be labelled by enzymatic incorporation of modified nucleic acid or conjugation of aliphatic amines to 3'- or 5'-phosphates. Details are given in Haugland R.P. "Handbook of Fluorescent Probes and Research Chemicals" *supra.* Green fluorescent protein mutants are encoded in plasmid DNA as fusion constructs with a protein of interest and expressed by introduction of the plasmid in cells.

In step (1) of the method of the invention a sample is provided which comprises the macromolecular component bound to the donor fluorophore. This sample may be prepared in any suitable way. For example, when the sample is a cell, the macromolecular component may be bound to the donor fluorophore by introducing into the cell a moiety which is linked to the donor fluorophore and which binds selectively and tightly to the macromolecular component. For example, a donor fluorophore-labelled antibody which is selective for the macromolecular component may be introduced into the cell. As a further example, the donor fluorophore may be linked to a selective, tight-binding ligand of the macromolecular component which, when introduced into the cell, binds to the macromolecular component. Suitable ligands are well known for many macromolecular components; for example, many enzymes bind tightly and selectively to inhibitor molecules which themselves may be fluorescent, or may be bound to a suitable donor fluorophore. The moiety which is linked to the donor fluorophore may be introduced into the cell using any suitable method including, for example, microinjection, electroporation, use of cell permeable delivery systems such as liposomes, or using protein delivery systems such as the well known Penetratin *Drosophila* peptide, or functional equivalent thereof.

An alternative way of preparing a sample comprising the macromolecular component which is bound to a donor fluorophore, which is suitable when the macromolecular component is a protein, is to use genetic engineering methods. In particular, a polynucleotide encoding a fusion of the macromolecular component and an intrinsically fluorescent polypeptide such as a GFP may be constructed and introduced into the cell using well known methods, for example by using standard laboratory molecular biology methods such as those described in Sambrook *et al* (1989) *Molecular cloning, A laboratory manual,* Cold Spring Harbor Press, Cold Spring Harbor, New York.

Nucleic acid encoding the fusion may be used in accordance with known techniques, appropriately modified in view of the teachings contained herein, to construct an expression vector, which is then used to transform an appropriate host cell for the expression and production of the fusion. Such techniques include those disclosed in US Patent Nos. 4,440,859 issued 3 April 1984 to Rutter *et al*, 4,530,901 issued 23 July 1985 to Weissman, 4,582,800 issued 15 April 1986 to Crowl, 4,677,063 issued 30 June 1987 to Mark *et al*, 4,678,751 issued 7 July 1987 to Goeddel, 4,704,362 issued 3 November 1987 to Itakura *et al*, 4,710,463 issued 1 December 1987 to Murray, 4,757,006 issued 12 July 1988 to Toole, Jr. *et al*, 4,766,075 issued 23 August 1988 to Goeddel *et al* and 4,810,648 issued 7 March 1989 to Stalker.

Polynucleotides, such as DNA, encoding the fusion may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transariptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Many expression systems are known, including bacteria (for example *E. coli* and *Bacillus subtilis*), yeasts (for example *Saccharomyces cerevisiae*), filamentous fungi (for example *Aspergillus*), plant cells, animal cells and insect cells.

The vectors include a prokaryotic replicon, such as the ColE1 *ori*, for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and pTrc99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps).

A variety of methods have been developed to operably link DNA to vectors via complementary cohesive termini. For instance, complementary homopolymer tracts can be added to the DNA segment to be inserted in to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion is treated with bacteriophage T4 DNA polymerase or *E. coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the fusion is to use the polymerase chain reaction as disclosed by Saiki *et al* (1988) *Science* **239,** 487-491. In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

Transformation of appropriate cell hosts with a nucleic acid encoding the fusion is accomplished by well known methods that typically depend on the type of vector used. With regard to transformation of prokaryotic host cells, see, for example, Cohen *et al* (1972) *Proc. Natl. Acad. Sci. USA* **69,** 2110 and Sambrook *et al* (1989) *Molecular Cloning, A Laboratory Manual,* Cold Spring Harbor Laboratory, Cold Spring Harbor, NY. Transformation of yeast cells is described in Sherman *et al* (1986) *Methods In Yeast Genetics, A Laboratory Manual,* Cold Spring Harbor, NY. The method of Beggs (1978) *Nature* **275,** 104-109 is also useful. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming cells and is well known in the art for transforming yeast cells, bacterial cells and vertebrate cells.

For example, many bacterial species may be transformed by the methods described in Luchansky *et al* (1988) *Mol. Microbiol.* **2**, 637-646. The greatest number of transformants is consistently recovered following electroporation of the DNA-cell mixture suspended in 2.5X PEB using 6250V per cm at 25:FD.

Methods for transformation of yeast by electroporation are disclosed in Becker & Guarente (1990) *Methods Enzymol.* **194,** 182.

Successfully transformed cells, ie cells that contain a DNA construct of the present invention, can be identified by well known techniques. For example, cells resulting from the introduction of an expression construct of the present invention can be grown to produce the polypeptide of the invention. Cells can be harvested and lysed and their DNA content examined for the presence of the DNA using a method such as that described by Southern (1975) *J. Mol. Biol.* **98,** 503 or Berent *et al* (1985) *Biotech.* **3,** 208. Alternatively, the presence of the protein in the supernatant can be detected using antibodies as described below.

In addition to directly assaying for the presence of recombinant DNA, successful transformation can be confirmed by showing that it expresses the fluorescent molecule

It will be appreciated that in certain embodiments of the invention the sample may be a sample which contains the macromolecular component, but that the sample is not, and does not contain, live, or intact cells. For example, the sample may be an historic sample such as cells fixed in paraffin section, or it may be a sample in which the macromolecular component is in a form isolated from other cellular molecules (eg isolated from a cell, or produced by genetic engineering means) such as may be used in a high throughput drug screen. In these cases, the sample may be prepared in any suitable way, for example by treating a paraffin section of a tissue sample in the usual way for binding of antibodies (which may include microwave irradiation) and contacting the so-prepared sample with an antibody (or other binding moiety) which has been labelled with the donor fluorophore, or for example by preparing a fusion of the macromolecular component and the donor fluorophore (for example, by genetic engineering means).

In step (2) the sample provided in step (1) is contacted with a sensor molecule which is bound to an acceptor molecule. Any suitable method of contacting the sensor with the sample may be used, and similar considerations as to suitability apply as in the preparation of the sample of step (1). It will be appreciated that when the donor fluorophore is bound indirectly to the macromolecular component as described above, appropriate modifications to the way the sample is prepared may be needed. For example, the further moiety which links the macromolecular component to the donor fluorophore may be introduced into the cell at an appropriate time. Similar considerations apply to the situation where the sensor does not bind directly to the second state of the macromolecular component.

In a further embodiment of the invention, the sensor molecule is included in a library of molecules some of which may be sensor molecules, but most of which are not sensor molecules. The sensor molecules and the other molecules in the library are bound to an acceptor molecule and in step (2) of the method of the invention individual members of the library are contacted with the sample as given. Those molecules which are sensor molecules present in the library are those for which FRET is detected by the method of step (3). It will be appreciated that this embodiment may be used to detect new sensor molecules. For example, when the molecular component is a protein phosphorylated in a particular way, the method may be used to detect sensor molecules which bind to the phosphorylated protein but not to the non-phosphorylated protein.

Typically, in this embodiment, a nucleic acid library encoding potential sensors is made, and individual nucleic acid molecules which encode a potential sensor are introduced into an appropriate cell as in step (2).

The methods of the invention may be used in many areas of biology and biological research including drug screening, diagnostics and academic research.

It will readily be appreciated that the methods may be used for screening for compounds which modulate the interconversion of the macromolecular component from a first state to a second state. For example, the methods may be used for identifying compounds which inhibit or enhance phosphorylation of a particular protein under a given condition. Suitable formats and controls for high throughput screening will be apparent to the person skilled in the art. It will be appreciated that there are a number of advantages of the methods over conventional drug screening methods. For example, the methods allow screening to be carried out in living cells in real time so that rapid changes in the state of the macromolecular components upon contact of the cells with potential drugs or drug-like compounds may be detected. A further advantage is that, at least in one embodiment, the method can be used to spatially resolve the fluorescence lifetime measurement. A practical effect of this is that when the drug screening methods is carried out in an array format (which is frequently the case, such as in microtitre plates) the read-out from each well is spatially resolved so all of the wells can be monitored in one go thus increasing the rapidity of screening.

There is an increasing use of miniaturisation in high throughput drug screening assays, driven by the desire to reduce the quantity of expensive reagents used. Thus, it is now possible for nl to μl amounts of reagent solutions to be used, which leads to difficulties in pipeting small volumes. Since an excess of the sensor does not affect the methods of the invention, at least in respect of the sensor, control of volume is much less critical than in other drug screening systems where concentrations of reagents may be critical. In addition, donor lifetimes are independent of probe concentration and light path length which again does not necessitate tight control of sample concentration, sample volume and sample dimensions or geometries. Also, in the quest for miniaturisation, higher and higher density arrays, some of which have microchannels allowing mixing of small volumes of reagents, or allowing small volumes of reagents to contact a cell sample are being used, and the ability to use the methods of the invention to spatially resolve at very high density is advantageous.

A further use of the methods of the invention is in diagnostic methods. The methods may be used to analyse samples (which may be historic samples) from a patient and to determine the state of particular macromolecular components. For example, the phosphorylation states of particular proteins may be determined in cells from a patient using the methods of the invention, and this information may be useful in aiding diagnosis or in determining an appropriate treatment regime. As a further example, the methods of the invention may be used to determine, in a patient who is being treated with a particular drug, what effect that drug is having on the state of a particular macromolecular compartment within a cell from the patient. In this example, an analysis of changes the drug has made to various biochemical pathways can be undertaken by determining the state of various macromolecular components of the pathway. Similarly, the methods can be used to determine which biochemical pathways are active or not active in a particular disease by analysing the states of various macromolecular components in the biochemical pathways in cells from a diseased individual.

In a preferred embodiment of the invention, which is described in more detail in the Examples the phosphorylation state of threonine 250 on protein kinase C $\alpha$ (PKC$\alpha$), which is a marker for the activation of the protein, is determined. By using a GFP-fusion construct of PKC$\alpha$ the phosphorylation state and thereby the activation state of the protein can be followed by measuring the fluorescence lifetime of GFP at each pixel with FLIM in live cells injected with Cy3 labelled Fab or Cy3 labelled antibodies against phosphorylated T250. In fixed tissues the activation state of PKC$\alpha$ can be determined by using a reporter antibody (MC5) against the hinge region of PKC$\alpha$ with Cy3 as a donor fluorescent group, and a sensor antibody against phosphorylated T250 labelled with Cy5 as an acceptor group.

In another preferred embodiment, the phosphorylation state of tyrosines on the EGF receptor, and thereby its activation state, can be determined in pathological tissues or single cells.

A further aspect of the invention provides a kit of parts comprising (a) a macromolecular component covalently linked to a donor fluorophore or a nucleic acid molecule encoding a macromolecular component protein fused to a donor fluorophore protein and (b) a sensor molecule covalently linked to an acceptor molecule or a nucleic acid molecule encoding a sensor protein fused to an acceptor protein, wherein the macromolecular component can exist in a first state or a second state which differ by virtue of a covalent modification or which are different conformations wherein in its first state the macromolecular component is substantially incapable of being bound by the sensor molecule and in its second state the macromólecular component is able to be bound by the sensor molecule.

Kit of parts may be readily designed based on the components described above for use in the methods of the invention.

It is particularly preferred if the macromolecular component is a protein and that it is fused to a GFP. Such fusions may conveniently be encoded and expressed by nucleic acid constructs as described above.

It is also particularly preferred if the sensor molecule is one which can detect covalent changes in the macromolecular component. For example, it may be an antibody which can detect phosphorylation of a protein.

A particularly preferred kit of the invention is a nucleic acid encoding a phosphorylatable protein (such as protein kinase C) fused to a donor green fluorescent protein, and an antibody bound to an acceptor molecule which antibody binds to the phosphorylated protein but not to the non-phosphorylated protein.

A suitable antibody is one which binds a phosphorylated amino acid residue (eg phosphoserine, phosphothreonine or phosphotyrosine). The antibody may also be one which binds specifically to a particular phosphorylated site on a protein. For example, the antibodies which recognise specifically phosphorylated T250 in PKC$\alpha$, as described in the Examples, are such antibodies.

The invention will now be described in more detail by reference to the following Figures and Examples where:

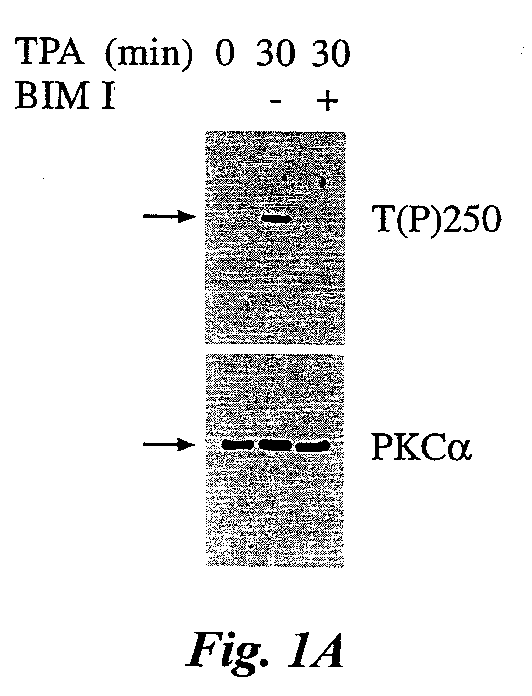

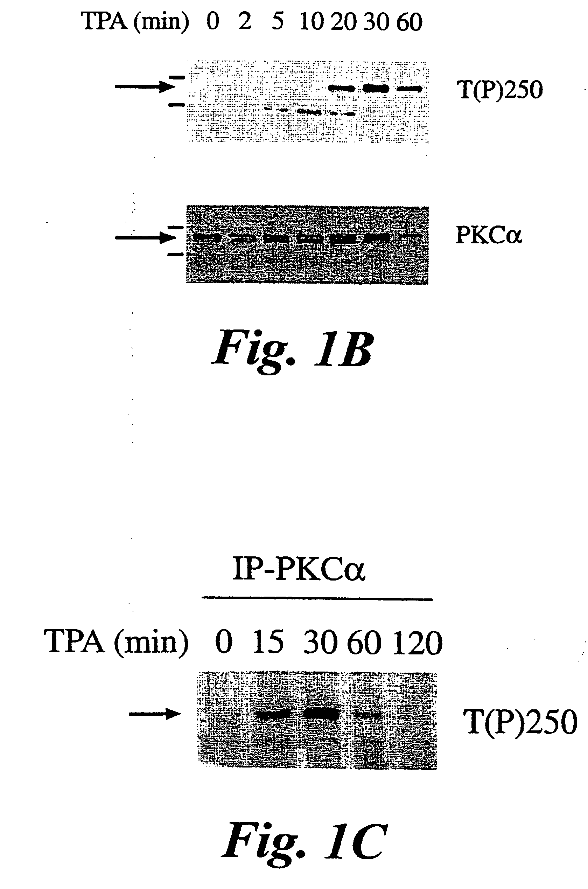

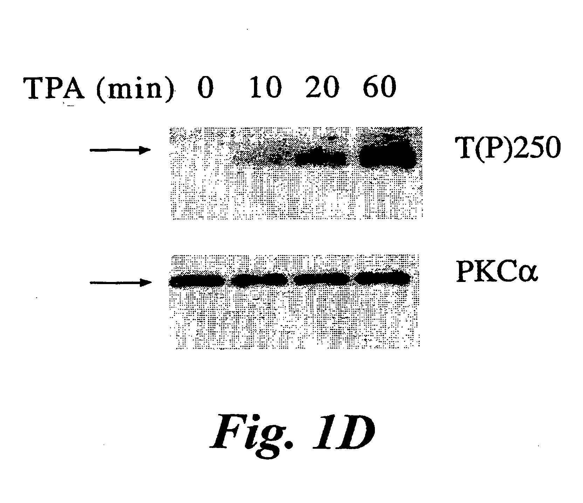

**Figure 1. Identification of an autophosphorylation site on PKC$\alpha$.**

**A**. COS-7 cells were transfected with a cDNA expression plasmid for PKC$\alpha$. After 48h, the cells were treated with TPA for 0 or 30 minutes either in the presence (+) or absence (-) of the PKC inhibitor bisindolylmaleimide I (10μM).

The upper panel indicates immunoreactivity with the T(P)250 antiserum (arrow)[34]. The lower panel shows immunoreactivity with an antibody against PKCα protein (MC5; arrow).

**B.** Transfected COS-7 cells expressing PKCα were treated for the times indicated with TPA (400nM). Extracts were prepared and subjected to Western blotting. The upper panel shows the immunoreactivity for the T(P)250 antibody and is indicated by an arrow. The lower panel shows that there was no change in PKCα (arrow) during this treatment until the 60 minute time point, where downregulation is evident.

**C**. PKCα from transfected COS-7 cells treated with TPA (400nM) for the times indicated, was immunoprecipitated from extracts with MC5[9] and subjected to Western blotting with the T(P)250 antibody. Immunoreactive protein is indicated by the arrow. As observed in whole cell extracts, the induced phosphorylation was reduced at later times, due to downregulation.

**D**. COS-7 cell expressed, his-tagged PKCα was purified by nickel-agarose chromatography[10]. Autophosphorylation of purified PKCα was carried out in the presence of TPA (2µM), phosphatidylserine (1.25mg/ml) in 1% (v/v) Triton X-100, 50 mM Hepes pH7.5, 12.5 mM $MgCl_2$ and 100 µM ATP. Reactions were initiated with ATP and terminated with Laemmli sample buffer at the times indicated. Phosphorylation of the T250 site was monitored with the T(P)250 antiserum (upper panel) and the amount of PKCα protein with MC5 (lower panel). Immunoreactive proteins are indicated with arrows.

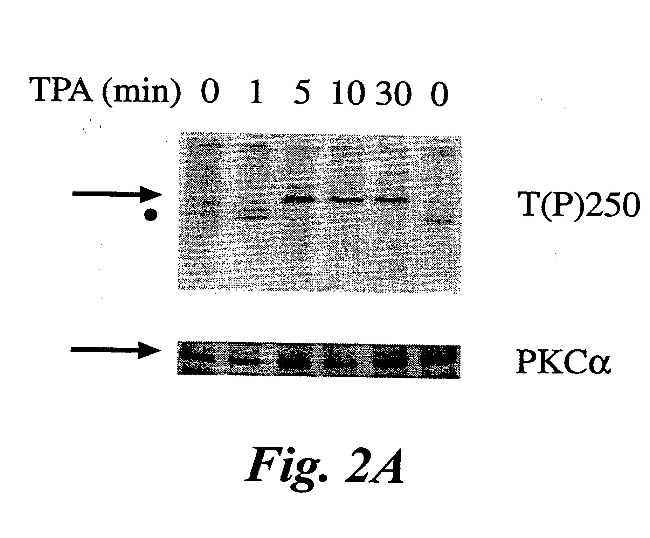

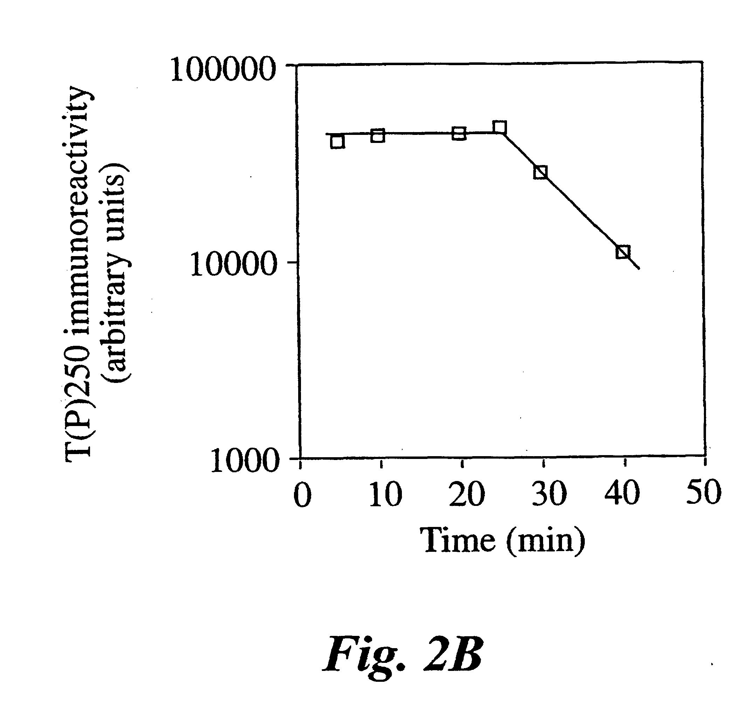

**Figure 2. Use of autophosphorylation site specific antisera to monitor PKC activation by Western blotting.**

**A**. Quiescent Swiss 3T3 cells (see [35] were treated with TPA for the times indicated, or left untreated (lanes marked '0' ). Extracts were prepared and blotted for immunoreaction with the T(P)250 antibody (arrow, upper panel) or for PKCα protein (arrow, lower panel). The dot in the upper panel indicates an immunoreactive band that is variably observed and not competed by the phospho-peptide antigen (data not shown).
**B**. Quiescent 3T3 cells were treated with PDBu (500nM) for 20 minutes and then washed with medium at 4°C and incubated at 37°C in the absence of PDBu for the times indicated. Cell extracts were blotted with the T(P)250 antiserum and immunoreaction quantified by scanning densitometry. The graph illustrates the lag period observed prior to an exponential decay in T(P)250 immunoreaction.

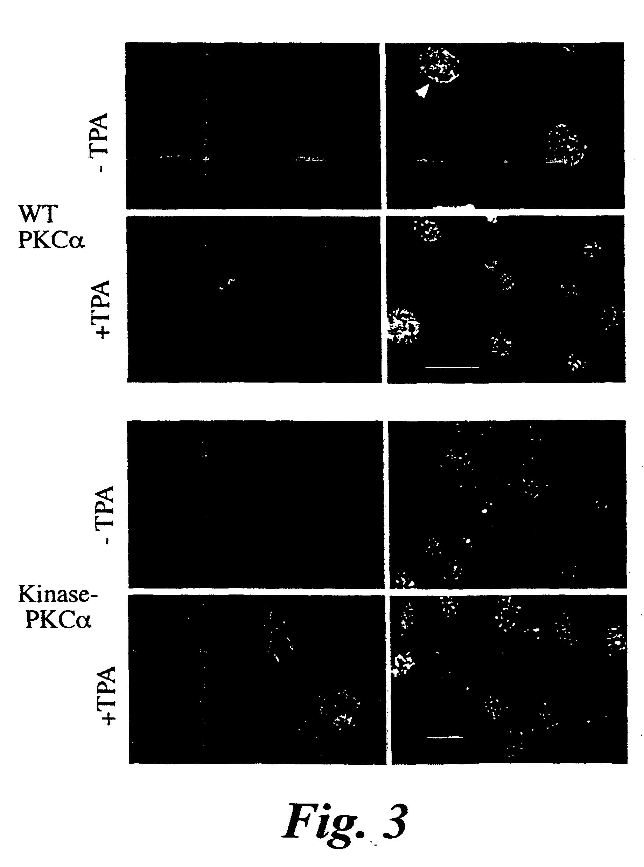

**Figure 3. Detection of PKCa activation in transfected cells.**

Upper panels, COS-7 cells transiently transfected with a *myc*-tagged PKCα construct were untreated (con) or treated for 20 minutes with TPA (400nM) as indicated. Following treatment cells were fixed and stained for myc-PKCα (monoclonal 9E10) or for T(P)250. In addition to activated PKCα, the latter revealed a non-PKCα nuclear reaction in both untransfected (arrowhead) and transfected cells (bar = 25µm). Lower panel, plasma membrane translocation of kinase inactive PKCα which did not undergo T250 phosphorylation in response to TPA (400nM). Transiently transfected cells were fixed and co-stained with MC5 and the T(P)250 serum (bar = 100µm).

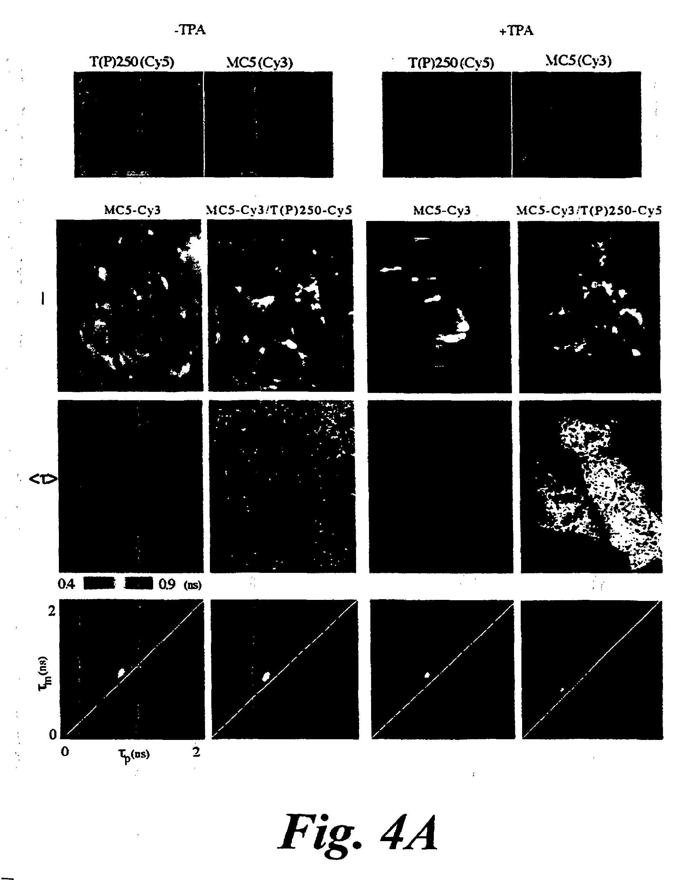

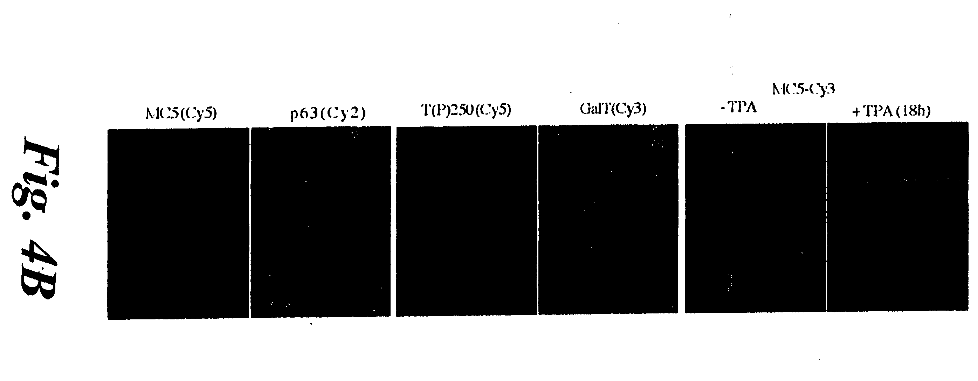

**Figure 4. Endogenous PKCα activation detected by immunofluorescence and FLIM.**

**A.** Upper panels, stacked confocal micrographs showing an increase in T250 phosphorylation of endogenous PKCα following 20 min of TPA stimulation (+TPA), as detected by a Cy5-conjugated IgG fraction of T(P)250 (T(P)-IgG-Cy5). There was no apparent difference in the level of PKCα protein expression, before and after TPA treatment, as demonstrated by concomitant detection with MC5-Cy3. Double-label immunofluorescence staining was performed as described[36] except for minor modifications[37]. Antibodies were conjugated to fluorophores as previously[38]. Middle panels: I shows the steady-state fluorescence images from the donor fluorophore (Cy3) conjugated to MC5 in fixed 293 cells which were stained with either MC5-Cy3 alone or with both MC5-Cy3 and T(P)250-IgG-Cy5 (acceptor fluorophore) as indicated[39]. FRET from the donor (Cy3) to acceptor (Cy5) fluorophore took place on individual PKCα molecules which were phosphorylated after TPA stimulation, resulting in a reduction of the fluorescence emission lifetime of Cy3 as demonstrated by changes in <τ> (the average of $\tau_p$ and $\tau_m$). The 2D histograms in the lowest panels quantify the reduction of the donor lifetimes of Cy3 emission for both the phase ($\tau_p$) and modulation ($\tau_m$). A detailed description of the FLIM apparatus used for FRET determination can be found elsewhere[21].
**B.** The panels show stacked confocal micrographs for PKCα (MC5 or T(P)250) and ER (p62) or Golgi (GalT) markers as indicated. Right-hand panels, demonstrate the specificity of MC5 immunodetection by downregulation

of PKC$\alpha$ following prolonged (18h) exposure of 293 cells to TPA (1$\mu$M).

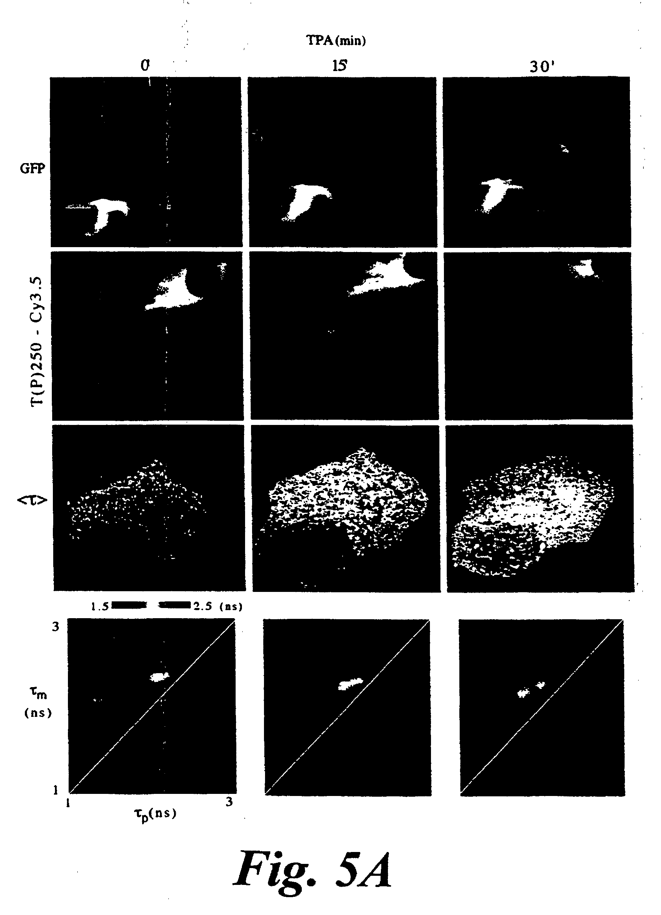

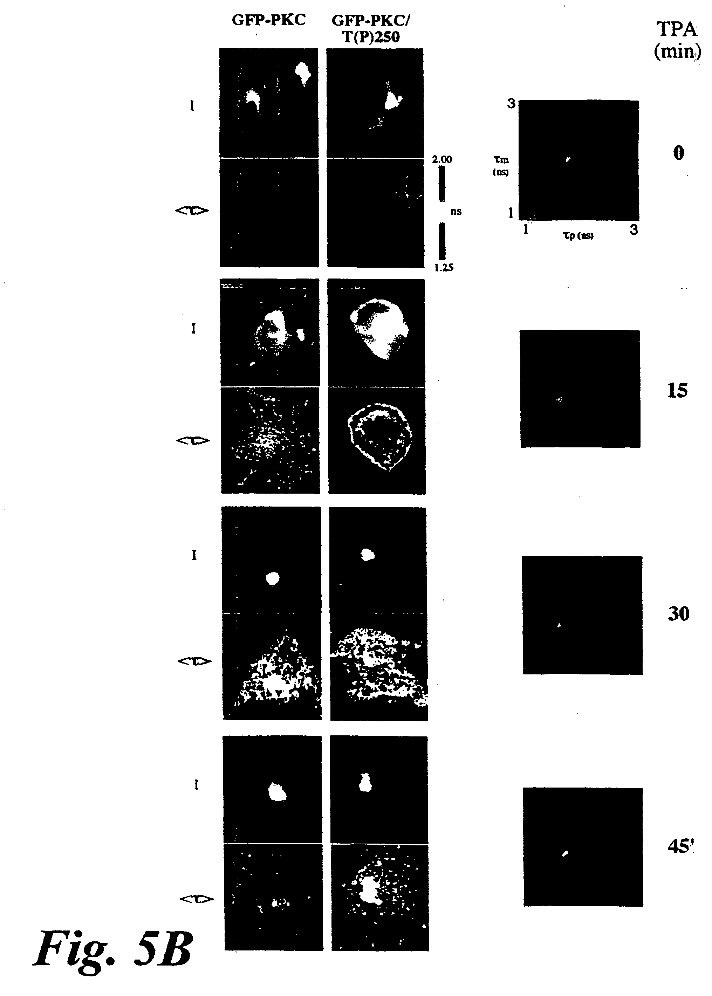

**Figure 5. TPA-induced Thr-250 phosphorylation of GFP-tagged PKC$\alpha$ in live COS-7 cells detected by FLIM.**

**A.** COS-7 cells were transiently transfected with an N-terminal GFP-tagged PKC$\alpha$ construct and cultured in serum-containing DMEM for 36h before transfer to serum- and phenol red-free Optimax medium (Life Technologies). After an overnight culture, cells were washed and resuspended in PBS, then microinjected with a Cy3.5-conjugated, protein G-purified IgG fraction of T(P)250 {T(P)250-IgG-Cy3.5}. GFP, the fluorescence images from the donor (GFP) in four live COS-7 cells which were either left as controls or microinjected with T(P)250-IgG-Cy3.5. The bottom four cells were transfected but only the top three cells (including an untransfected cell) were microinjected with T(P)250-IgG-Cy3.5. By 30 min of TPA stimulation, two clearly separate fluorescence lifetime populations belonging to the microinjected and uninjected cells, respectively, were evident from changes in both the phase ($\tau_p$) and modulation ($\tau_m$) lifetimes of GFP emission (bottom panels). Figure 5A is representative of five independent experiments. A detailed description of the FLIM apparatus used for FRET determination in can be found elsewhere[21].
**B.** COS-7 cells were transiently transfected with a GFP-tagged PKC$\alpha$ construct, stimulated with TPA (400nM) and fixed at various time points as indicated, then either left as controls (GFP-PKC) or stained with T(P)250-IgG-Cy3.5 (GFP-PKC/T(P)250). The fluorescence images from the donor (I) are shown. The cumulative lifetimes of GFP-PKC alone (green) and that measured in the presence of the acceptor fluorophore (T(P)250-IgG-Cy3.5) (red) are plotted on the same 2D histogram for each time point.

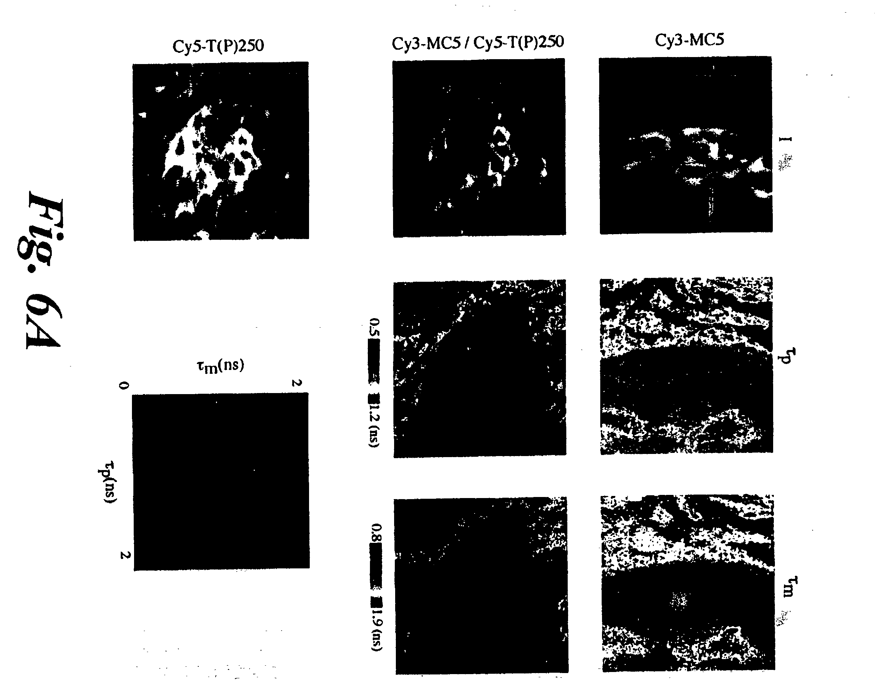

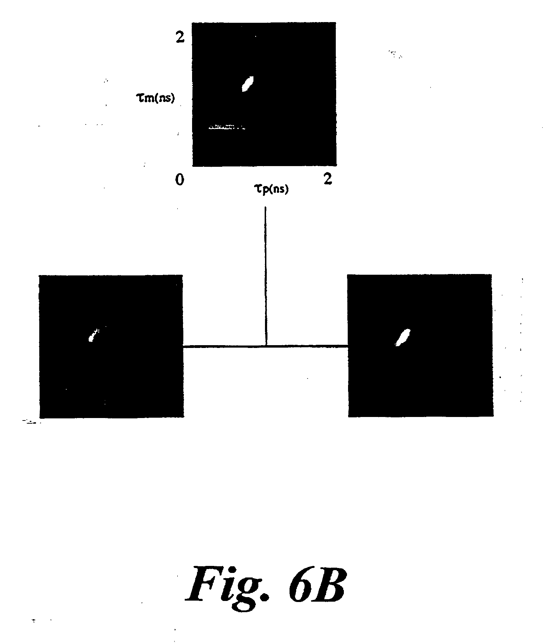

**Figure 6. Demonstration of a variable degree of Thr-250 phosphorylation of PKC$\alpha$ in paraffin-embedded, fixed tissue sections of human breast carcinomas.**

**A.** Paraffin-embedded breast cancer sections were dewaxed in xylene, pressure cooked for antigen-retrieval[40], then stained with either MC5-Cy3 alone or with both MC5-Cy3 and T(P)250-IgG-Cy5. The left-hand panels show the donor (upper and middle) and acceptor (lower) fluorescence patterns. The corresponding lifetime images for the upper two sections are shown on the right. The 2D histogram (bottom right) shows the donor lifetimes ($\tau_p$ and $\tau_m$) from these images.
**B.** The histograms shown, are derived from 23 patient samples. The upper panel shows the lifetimes ($\tau_p$ and $\tau_m$) from all patients. The lower panels illustrate those patient samples where PKC$\alpha$ is activated (left) and those where it is not (right); see text.

**Figure 7. Two ways of measuring phosphorylation of proteins in cells by detecting FRET through the lifetime decrease of the donor fluorophore on the reporter.**

(A) A GFP-fusion protein is the reporter containing the donor fluorophore GFP. Phosphorylation is detected via the ensuing FRET signal after binding of an antibody labelled with an acceptor fluorophore to the phosphopeptide on the reporter. (B) A specific antibody which binds to a protein is labelled with a donor fluorophore. Phosphorylation is detected via the ensuing FRET signal after binding of an antibody labelled with an acceptor fluorophore to the phosphorylated residue on the antibody-protein (reporter) complex.

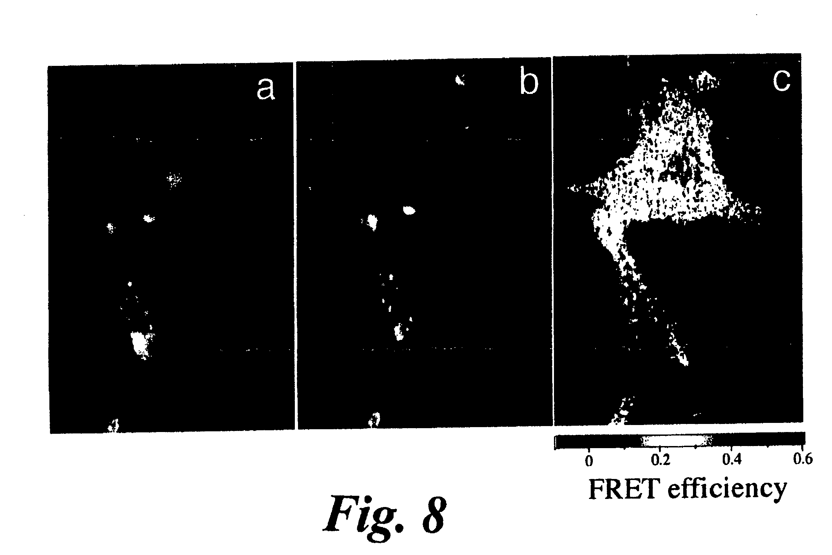

**Figure 8. Epidermal growth factor (EGF) induced phosphorylation of green fluorescent protein (GFP)-tagged EGF receptor ErbB1 in NR6 cells detected by fluorescence lifetime imaging microscopy (FLIM).**

Serum-starved cells were incubated for 10 min with 100 ng ml$^{-1}$ EGF and fixed in 4% paraformaldehyde. (a) Fluorescence image of GFP-ErbB1; (b) fluorescence image of phosphotyrosine-specific antibody PY-72-Cy3; (c) fluorescence resonance energy transfer (FRET) efficiency map calculated from the fluorescence lifetimes. See Example 2 for further details.

**Figure 9. Depiction of various scenarios for using the methods of the invention.**

MC =     macromolecular component
D =       donor fluorophore
A =       acceptor molecule
S =       sensor
X =       no binding of sensor to first state of macromolecular component

   (a) Covalent modification of first state MC to give second state.

   (b) Conformation change in MC from first state to second state.

   (c) Binding of further moiety to change first state of MC to second state.

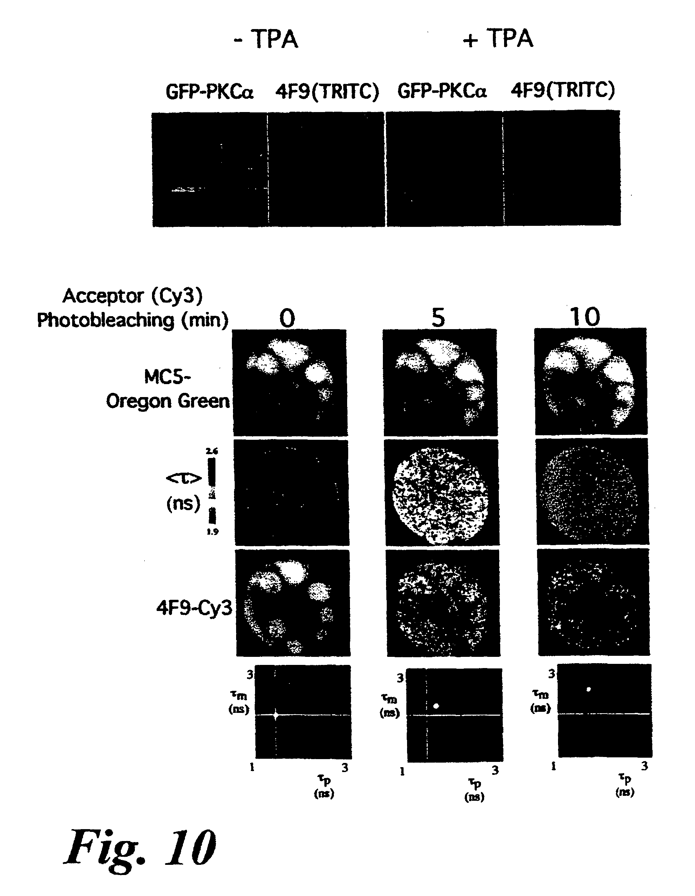

**Figure 10. Upper panel. 4F9 F(ab')2 fragments recognize the activated form of PKC$\alpha$ expressed in MCF-7 cells**

MCF7 cells were transiently transfected with a GFP-tagged PKC$\alpha$ construct, stimulated with TPA (400nM) and fixed after 20 min of incubation at 37°C. Cells were stained with F(ab')2 fragments of the anti-Thr 250 phosphospecific mAb 4F9 (IgM) followed by a TRITC-labelled anti-mouse Fab conjugate (Sigma). GFP-PKC$\alpha$ is recognized by the 4F9 F(ab')2 fragments only after activation by TPA (+TPA), following its translocation to the plasma membrane. The 4F9 monoclonal was generated by immunisation of mice with a phosphopeptide as described for polyclonal antisera (Ng *et al* (1999) *Science* **283,** 2085-2089). Standard procedures were employed to generate hybridomas and positives identified by ELISA. The 4F9 line was derived from a single cell clone and isotyped as an IgM. To provide good signal-to-noise, the IgM was cleaved with pepsin to generate F(ab')2 fragments.
**Lower panel. Employment of 4F9 F(ab')2 fragments to monitor the degree of PKC$\alpha$ activation in MCF10A cells by the detection of fluorescence resonance energy transfer (FRET).**
MCF-10A cells grown in Matrigel were costained with Oregon Green-labelled pan-PKC$\alpha$ mAb MC5 (MC5-Oregon Green) and Cy3-labeled 4F9 F(ab')2 fragments post-fixation. There is a significant degree of colocalisation between the intracellular staining of endogenous PKC$\alpha$ by MC5 and that of the phosphospecific mAb 4F9 F(ab')2 fragments. The molecular proximity between these two antibodies (ie their binding to the same PKC$\alpha$ molecule) was established by the presence of fluorescence resonance energy transfer (FRET) from Oregon Green to Cy3 in these cells. The specificity of this FRET was monitored by photobleaching the Cy3 acceptor, resulting in a lengthening of the average donor lifetime $<\tau>$ (($\tau_m + \tau_p$)/2) after photobleaching. The pseudocolour lifetime maps and 2D histograms clearly demonstrates the lengthening of the donor lifetime after photobleaching the acceptor.

**Example 1: Imaging protein kinase C$\alpha$ activation in cells**

*Summary of Example*

The determination of protein activity within the physiological context of the cell, is vital for obtaining true insights into the operation of signal transduction processes. Spatially resolved fluorescence resonance energy transfer (FRET) measured by fluorescence lifetime imaging microscopy (FLIM), provides not only a unique method for tracing the catalytic activity of fluorescently tagged signalling proteins inside living cells, but also the ability to determine the functional state of proteins in fixed cells and tissues. Here we exploit an autophosphorylation site (threonine 250) in the lipid/calcium-dependent protein kinase C$\alpha$ (PKC$\alpha$) as a dynamic marker of PKC$\alpha$ activation. The autophosphorylation of threonine 250 in PKC$\alpha$ is detected through the binding of fluorescently tagged phosphorylation site-specific antibodies; the consequent FRET is measured exclusively through the donor fluorophore on PKC$\alpha$ itself by FLIM. This approach enabled the imaging of PKC$\alpha$ activation in live and fixed cultured cells, demonstrating the compartmental nature of this response. The method was also applied to pathological samples where it is shown that a substantial proportion of breast tumours display activated PKC$\alpha$.

*Introduction*

The exquisite spatial resolution of biological processes afforded by selective interactions of proteins with other proteins, membranes, oligosaccharides or polynucleotides, has posed formidable problems in analysing protein behaviour in intact cells. The dynamic location of proteins has been monitored through various techniques including the recent widespread use of GFP-tagged proteins[1]. However for many proteins, there is a need to integrate the spatial data with information on catalytic function. This is a particular concern in signal transduction processes, where the networking of multiple inputs affects the output, leading to grossly different cellular consequences. Methods applicable to the functional analysis of particular (signalling) proteins *in situ* would provide a significant advance in reaching a molecular description of cellular behaviour.

Second messenger-dependent signal transducers characteristically undergo conformational changes when activated. The classical and novel protein kinase isotypes (cPKC, nPKC) behave in this fashion in response to their second messenger, diacylglycerol (DAG)[2-4]. This c/n PKC activator, as a neutral lipid, is restricted to membrane compartments and the stable membrane associated, DAG-dependent complexes formed by PKC have traditionally provided a useful means of monitoring PKC isotype activation (see[5]). However, PKC isotypes can be found associated with membranes prior to any exogenous stimulus and increasingly it is apparent that membrane versus non-membrane association is an insufficient criterion for determining PKC activation and certainly inadequate to determine its catalytic output[6]. This limitation is coupled to a more profound one, that is the inability to assess the activation status of PKC (and many other signal transducers) *in situ*, so allowing definition of temporo-spatial activation in live or fixed samples. In particular an ability to determine the degree of PKC activation in human pathological specimens would provide a rational basis for investigating the use of newly developed pharmacological inhibitors (recently reviewed in[7]).

Using fluorescence lifetime imaging microscopy (FLIM), we describe the activation state of PKC$\alpha$ *in situ.* The power of this approach is exemplified in analyses of fixed and live cells and additionally of archived human tissues.

*An activation marker for cPKC isotypes*

An *in vivo* phosphorylation site in PKC$\alpha$ has been identified as threonine 250[8]. To confirm this assignment and to monitor site occupancy, antibodies were raised against the cognate phosphorylated oligopeptide. As illustrated in Figure 1A, the site-specific antiserum against phosphorylated threonine 250 (T(P)250) reacted with PKC$\alpha$ expressed in COS-7 cells only following direct stimulation with tetradecanoyl phorbol acetate (TPA). In the presence of the PKC inhibitor bisindolylmaleimide I, no increase in immunoreaction was observed in response to TPA. The increase in immunoreaction following TPA treatment was time-dependent, with optimum phosphorylation induced at 30 minutes with 400nM TPA (Figure 1B). There is a decrease in immunoreaction at 60 minutes, but this parallels the TPA-induced loss of PKC$\alpha$ protein (see Figure 1B lower panel). To confirm that the T(P)250 immunoreactive protein observed is PKC$\alpha$, extracts were immunoprecipitated with the MC5 monoclonal antibody[9] prior to Western analysis. The protein recovered from COS-7 cells displayed a TPA-induced, time-dependent increase in T(P)250 immunoreaction (Figure 1C).

The behaviour of the T250 site suggests that this is an agonist-sensitive autophosphorylation site. Consistent with this, purification of His-tagged PKC$\alpha$ from unstimulated COS-7 cells[10] followed by an *in vitro* autophosphorylation reaction in the presence of phospholipid/TPA and Mg-ATP, led to a time-dependent increase in immunoreaction with the T(P)250 antiserum (Figure 1D). These results demonstrate that the T250 site is an autophosphorylation site *in vitro* and *in vivo.* The equivalent site in PKC$\beta$ is also an autophosphorylation site; on incubation with phospholipid/TPA and Mg-ATP, purified PKC$\beta$1 becomes immunoreactive with the T(P)250 antiserum. This site is also conserved in PKC$\gamma$ but not in other PKC isotypes (data not shown).

To assess the sensitivity of T(P)250 immunoreaction, the response of endogenous PKC$\alpha$ was determined in a well characterised cell line, Swiss 3T3 cells. These cells can be induced to quiesce and display a spectrum of well documented responses on subsequent hormone or growth factor stimulation[11]. Exposure of quiescent Swiss 3T3 cells to TPA induces the rapid phosphorylation of PKC$\alpha$ (Figure 2A). This is time-dependent with optimum phosphorylation induced by five minutes.

The use of the T250 autophosphorylation site as a dynamic marker of activation is dependent on the rate of phosophate turnover in the cell. Thus, the dynamics of dephosphorylation were monitored upon the removal of the relatively hydrophilic agonist, phorbol dibutyrate (PDBu). Following a lag period of variable length (over all experiments this was between 10 and 25 minutes), that probably reflects the re-equilibration of cellular PDBu pools, T(P)250 immunoreaction decreased with a half-life of ~5 minutes (Figure 2B). Thus the dynamics of PKC activation can be followed through the immunoreaction of T(P)250.

*Spatial resolution of PKC$\alpha$ activation in fixed and live cells*

The ability to monitor the activation of PKC through its phosphorylation state provides a means of localising activated PKC, without the assumption that all membrane associated protein is ligand bound and catalytically competent. Transiently transfected COS-7 cells fixed and stained for PKC$\alpha$ show that in unstimulated cells, there is a heterogeneous cytoplasmic distribution of the protein with some concentration on (membranous) structures around the nucleus (Figure 3). On stimulation by TPA there is a redistribution of PKC$\alpha$ with an initial accumulation at the plasma membrane (see also below for a more detailed time course). In unstimulated cells, the T(P)250 antiserum stained only a nuclear antigen in a non-specific fashion as evidenced by immunoreaction with both transfected and non-transfected cells (upper right-hand panel). In stimulated cells, the T(P)250 antibody colocalised with both the membrane-associated and cytoplasmic PKCα, indicating phosphorylation of the protein. The same response was observed in both transiently transfected MCF-7 and microinjected Swiss 3T3 cells (data not shown). These responses are consistent with the Western analyses and indicate that the T(P)250-specific serum detects activated PKC in intact cells. To confirm the autophosphorylation nature of the response in intact cells, a kinase inactive PKCα (K368M kinase⁻ PKCα[12]) was transiently expressed and immunoreactivity compared between stimulated and unstimulated cells (Figure 3, lower panels). Only the non-specific nuclear staining was evident in these cells irrespective of TPA treatment. Thus the phosphorylation response requires an intrinsic catalytic function, consistent with the notion that this site is autophosphorylated.

To monitor the activation of endogenous PKCα, the fluorophore Cy3 was conjugated to MC5 (MC5-Cy3), and the fluorophore Cy5 was conjugated to purified T(P)250 immunoglobulin (T(P)250-IgG-Cy5). In unstimulated, fixed 293 cells, heterogeneous staining of the protein was observed with only weak immunoreaction with T(P)250-IgG-Cy5 (Figure 4A upper panels). On stimulation with TPA, T(P)250-IgG-Cy5 immunoreactivity was greatly increased and colocalised with MC5 immunoreactivity, consistent with activation of PKCα. Colocalisation gives only an indication of association, since the determination of proximity is limited by the resolution of the optical system. FRET between Cy3 and Cy5 measured by FLIM[13-21] provides a nm resolved, 'two-site' molecular proximity assay, for the quantitative determination of the activation and intracellular location of PKCα[22]. Lifetimes of the Cy3 donor fluorophore (conjugated to MC5) were monitored in cells stained with MC5-Cy3 alone or both MC5-Cy3 and T(P)250-IgG-Cy5. The Cy3 lifetimes (Figure 4A middle panels) displayed a marked reduction following TPA stimulation, when staining was performed with both antibodies; for MC5-Cy3 alone there was no change in lifetimes. The most dramatic reduction in lifetimes (from 0.9 to 0.4 ns) was associated with regions of the plasma membrane and vesicular structures in the cytoplasm. These PKCα-containing vesicular structures overlapped in part with the distribution of ectopically expressed PKCα described for NIH3T3 cells[23]. This pattern of behaviour reflects the activation of PKCα at the plasma membrane followed by internalisation and accumulation on large vesicular structures. Interestingly, the reduced lifetimes observed in the unstimulated cells were also localised to punctate cytoplasmic structures that would appear to reflect a basal activation and movement through a similar vesicular compartment. The 2-D histograms in the lower panels (Figure 4A), clearly illustrate the significant shift in both the phase ($\tau_p$) and modulation ($\tau_m$) lifetimes of the donor Cy3 emission (two independent measures of fluorescence lifetime[24]); this was observed on TPA treatment only when the acceptor fluorophore was also present (far right-hand panel).

To demonstrate the specificity of the FRET signals as measured by FLIM, cells were stained for PKCα or activated PKCα and in parallel for endoplasmic reticulum (anti-p62-Cy3) or Golgi (anti-galactosyl transferase-Cy3 or p115-Cy3). There is some overlap in localisation of PKCα with both compartments (Figure 4B), however there was no FRET between the fluorophores and thus no reduction in lifetime was observed (data not shown). Notably PKCα has been shown by electron microscopy to bind to both cis and trans Golgi regions[25]. Subsequent experiments in an independent cell line (COS-7 cells) have confirmed localisation of endogenous PKCα to both endoplasmic reticulum and the Golgi apparatus; the relative distribution between the two organelles appears to be cell density-dependent[26]. There was no significant overlap between vesicles containing endogenous T(P)250 immunoreactive PKCα and various membranous structures including early or late endosomes (no colocalization with Rab5 or Rab7), actin cytoskeleton (phalloidin staining), mitochondria (cytochrome oxidase IV subunit) or lysosomes (LysoTracker, Molecular Probes)[26]. A partial overlap was found however with structures containing caveolin (data not shown). The specificity of the PKCα antibody itself (MC5) is essential for the interpretation of the FLIM measurements. To demonstrate this specificity, cells were treated for 18h with TPA (1μM) in order to downregulate PKCα. Following treatment, immunoreactivity with MC5 was abolished (Figure 4B).

FRET imaging through donor photophysical properties alone has the advantage that the acceptor fluorophore is not detected and thus its concentration does not need to be known or controlled for in the experiment, whereas FRET efficiencies at each pixel can be derived easily[27]. It is this property of FLIM that can be exploited to analyse the activation of a donor-fluorophore tagged PKCα in live cells by microinjecting an excess of acceptor labelled T(P) 250-IgG. To achieve this, a GFP-PKCα fusion construct was prepared and transiently transfected into COS-7 cells. After 48h, some cells were microinjected with T(P)250-IgG-Cy3.5. Cultures were then monitored following treatment with TPA. The GFP-PKCα accumulated in a partly cytoplasmic and partly perinuclear location in untreated cells (upper panels in Figure 5A). Following TPA treatment of cells, there was a progressive increase in GFP-PKCα accumulation in vesicular structures within the cytoplasm. This was most evident after 30 minutes and observed in all four transfected cells visible in this field (Figure 5A upper panels). The two central GFP-PKCα transfected cells and one untransfected cell (Figure 5A second row) were microinjected with T(P)250-IgG-Cy3.5. As expected, the donor fluorophore (GFP) when visualised in the unstimulated cells was broadly dispersed in the cytoplasm and excluded from the nucleus. Analysis of mean fluorescence lifetimes (<τ>, the average of $\tau_p$ and $\tau_m$) reveals that there was a modest degree of GFP-PKCα phosphorylation in unstimulated cells, but following stimulation there was a progressive increase in T250 phosphorylation, evidenced by a decrease in GFP emission lifetimes; this was evident only in the two central GFP-PKC$\alpha$ transfected and T(P)250-IgG-Cy3.5 microinjected cells. At 15 minutes, the most intense decrease was observed in punctate areas adjacent to the plasma membrane. By 30 minutes, there was a more evident shift in donor emission lifetimes at the plasma membrane with a broader distribution of shorter lifetimes throughout the cytoplasm. It is notable that the microinjected cell in the upper right comer was not visible with the filter set used for GFP imaging, ie there was no bleedthrough emission detected emanating from the Cy3.5-labeled T(P)250 antibody. The $\tau_m$ and $\tau_p$ 2D-histograms are plotted to illustrate the clear temporal shift in lifetimes that occur on TPA stimulation (Figure 5A, lower panels). By 30 minutes there were two distinct lifetime populations within the field that represented the T(P)250-IgG-Cy3.5 microinjected cells and those expressing GFP-PKC$\alpha$ but not microinjected (cells in the lower left quadrant). Control experiments on fixed cells analysed by confocal imaging and also Western blotting, confirmed a redistribution of GFP-PKC$\alpha$ to a perinuclear compartment but no significant degree of protein degradation within this time frame (data not shown).

The pattern of PKC$\alpha$ behaviour in this live cell experiment illustrates the advantage of a direct activation marker over a simple localisation analysis. It is evident that activation is initiated at peripheral cell membranes. GFP-PKC$\alpha$ subsequently accumulates on vesicular cytoplasmic structures, however, these are not uniformly shifted in their fluorescence lifetimes. This indicates that while PKC$\alpha$ is activated and relocated to this vesicular compartment, the autophosphorylation in this compartment can be transient suggesting inactivation/dephosphorylation of the protein[28]. Nevertheless, monitoring of donor emission lifetimes in live cells for up to 40 min after TPA treatment showed that there was a persistent reduction of <$\tau$> in the transfected, microinjected cell population. In contrast, several control experiments carried out in fixed, GFP-PKC$\alpha$ transfected COS-7 cells clearly demonstrated a subsequent T250 dephosphorylation event after approximately 30 min of TPA treatment, with some degree of variation in time course among cells. An example is provided in Figure 5B which shows an initial membrane translocation of GFP-PKC$\alpha$ and an accompanying decrease in <$\tau$> (15 min TPA) in the transfected, T(P)250-IgG-labelled cell. This was followed by an almost complete redistribution of PKC$\alpha$ to a perinuclear compartment (30 min TPA) and a concomitant reversal of the initial reduction in <$\tau$>, indicative of dephosphorylation. The persistent reduction in <$\tau$> observed in live cells is probably due to a masking effect of the phosphospecific antibody which protects the epitope from subsequent dephosphorylation by phosphatases that act on PKC. This also shows that the FLIM experiments on live and fixed cells are complementary and need to be performed in parallel in order to get a comprehensive view on the time scale of phosphorylation/dephosphorylation of the PKC$\alpha$ T250 site.

*PKC$\alpha$ is activated in a subset of breast carcinomas*

Analysis of the activation status of signal transducers in fixed tissue samples would be a significant step in understanding the role of such proteins in human diseases. To establish the methodology, we have investigated the detection of activated PKC$\alpha$ in fixed tissue samples. This has involved analysis of a series of 44 formalin fixed paraffin-embedded breast tumour samples with confocal immunofluorescence imaging (using MC5-Cy3 and T(P)250-IgG-Cy5 conjugates); in a subset of these specimens (n=23), analysis of *in situ* PKC$\alpha$ phosphorylation by FLIM was performed. In these latter twenty three patients, parallel samples were stained either for PKC$\alpha$ (MC5-Cy3) or for both PKC$\alpha$ and T250 phosphorylation (T(P)250-IgG-Cy5). The results obtained fall into two categories, those patients where there was no detectable change in fluorescence lifetime (12/23) and those where there was a significant decrease in <$\tau$> (11/23). An example of the latter is shown in Figure 6A. Two different sections from one patient are shown. The upper panels represent an image of PKC$\alpha$ detected directly with MC5-Cy3, alongside the phase and modulation lifetime distributions for this field. Below are panels with the same information from a section labelled with both MC5-Cy3 and T(P)250-IgG-Cy5. It is clear from the pseudocolour images that the phase and modulation lifetimes were substantially reduced in the dual-labelled panels and that this reduction in lifetimes was restricted to the tumour mass (the background emission from the non-tumour areas of the section, serve to provide an internal control in the FLIM images; this does not display reduced lifetimes). The $\tau_p$ and $\tau_m$ decreased most strongly (blue) at the periphery of cells; this is most evident in the lower left tumour mass where the cells were outlined by the reduced $\tau_p$ and $\tau_m$ (Figure 6A, middle panels). Quantification of the $\tau_p$ and $\tau_m$ changes is provided by the 2D histogram in the bottom panel. Also shown (Figure 6B), are the pooled data from the two classes of patients (cumulative lifetime data of two fields for each of 23 patient samples). This illustrates those without any detectable change in fluorescence lifetime and those with a significant decrease in both $\tau_p$ and $\tau_m$. The results demonstrate that PKC$\alpha$ is activated in situ in a significant number of human breast tumours. Among the tumours where there was no evidence of T(P)250 immunoreactivity, some displayed an upregulation of PKC$\alpha$ protein as judged by MC5 immunoreaction. Thus increases in PKC content *per se* do not correlate with activation. This emphasises the need to be able to monitor the function of proteins for correlation with biological/pathological responses.

**EP 1 145 004 B1**

*Conclusions*

The results demonstrate that FRET measured with FLIM is applicable to detect catalytic functions *in vivo* as shown by the use of an autophosphorylation site in PKCα to monitor its activation. This autophosophorylation can be detected in fixed or live cells using a fluorescently labelled antibody specific for the occupied T250 site. FLIM provides a unique opportunity to follow the dynamics of this phosphorylation process in live cells through donor lifetime properties. Since the excess of microinjected acceptor labelled T250 antibody is not detected, the T250-IgG-Cy5 in the cell does not interfere with the measurement, a problem not soluble by conventional ratiometric FRET measurements.

The detection of autophosphorylation of PKCα on T250 itself has many advantages over the conventional activation determination, which is a cytosol versus membrane location assay. The PKC family of proteins are subject to control not only by effector binding, but also through phosphorylation, in particular in their activation loops[6]. This phosphorylation increases effector-dependent catalytic activity, a process not monitored by a simple evaluation of location (ie effector binding). Defining the extent of autophosphorylation of PKCα serves to assess its catalytic output directly and provides a very sensitive assay. The evidence provided here shows that in response to TPA, PKCα is activated at the plasma membrane and then traffics to intracellular vesicular compartments. This is not a peculiarity of TPA-induced activation, since in the absence of stimulation there is a basal degree of phosphorylation of PKCα similarly located. This tonic activation in 'unstimulated' cells represents a small population of PKCα that would not be observable by other criteria. Thus there is a degree of constitutive traffic of activated PKCα between the plasma membrane and these vesicular structures.

An upregulation of calcium-dependent PKC activity has been found in malignant tissues from breast cancer patients when compared to the normal breast tissues from the same individuals[29]. Additionally, PKCα has been implicated in the establishment of the multidrug resistant (MDR) breast tumour phenotype[30-33]. These observations were derived from either breast cancer cell lines or bulk tumour sample analyses. However, for effector-dependent signalling proteins such as PKCα, such changes in concentration do not necessarily reflect changes in function and may represent bystander effects. The use here of T(P)250 reagents and FLIM have allowed us to determine precisely the extent of PKCα activation *in situ* as well as its spatial distribution within tumour tissues. The data show that in fact there is no correlation between up-regulated expression and activation. Nevertheless a significant number of breast tumour samples (48%) show activated PKCα. To determine how PKCα activation may relate to prognosis, it will be important to analyse a larger cohort of patient samples and to correlate activation status with treatment and outcome.

***References to Example 1, including various methods***

1. R. Rizzuto, W. Carrington, R. A. Tuft (1998) *Trends Cell Biol* **8,** 288-92.
2. Y. Nishizuka (1986) *Science* **233,** 305-312.
3. H. Hug, T. F. Sarre (1993) *Biochem. J.* **291,** 329-343.
4. L. V. Dekker, P. J. Parker (1994) *Trends in Biochem. Sci.* **19,** 73-77.
5. A. S. Kraft, W. B. Anderson (1983) *Nature* **302,** 621-623.
6. J. A. Le Good, *et al* (1998) *Science* **281,** 2042-2045.
7. H. Hu (1996) *Drug Discovery Today* **1,** 438-447.
8. See the co-pending application GB 9803399.6.
9. S. Young, J. Rothbard, P. Parker (1988) *Eur. J. Biochem.* **173**, 247-252.
10. F. Bornancin, P. Parker (1996) *Curr. Biol.* **6,** 1114-1123.
11. E. Rozengurt (1986) *Science* **234,** 161-166.
12. C. Pears, P. J. Parker (1991) *FEBS Lett.* **284,** 120-122.
13. K. Carlsson, A. Liljeborg (1997) *J. Microsc.* **185,** 37-46.
14. C..Y. Dong, P. T. C. So, T. French, E. Gratton (1995) *Biophys. J.* **69,** 2234-2242.
15. T. W. J. Gadella, R. Clegg, D. J. Arndtjovin, T. M. Jovin (1993) *Biophysical Journal* **64,** A 130-A 130.
16. T. W. J. Gadella, T. M. Jovin, R. M. Clegg (1993) *Biophys. Chem.* **48,** 221-239.
17. J. R. Lakowicz, K. Berndt (1991) *Rev. Sci. Instrum.* **62,** 1727-1734.
18. R. Sanders, A. Draaijer, H. C. Gerritsen, P. M. Houpt, Y. K. Levine (1995) *Anal. Biochem.* **227,** 302-308.
19. P. C. Schneider, R. M. Clegg (1997) *Rev. Sci. Instrum.* **68,** 4107-4119.
20. P. T. C. So, *et al* (1995) *Bioimaging* **3,** 1-15.
21. A. Squire, P. I. H. Bastiaens (1998) *J. Microsc.* in press.
22. Following phosphorylation of PKCα, the molecular proximity (up to 7.5nm) of MC5-Cy3 and T(P)250-IgG-Cy5 (ie when bound to the same PKC molecule) leads to FRET from the donor to acceptor fluorophore and a consequent reduction in the fluorescence lifetime of the donor Cy3 molecule. Only the T(P)250-IgG-Cy5 antibodies which are in close molecular proximity to the MC5-Cy3 are detected through the reduction in fluorescence lifetime of the donor Cy3, whereas all the other acceptor T(P)250-IgG-Cy5 antibodies are not detected in the experiment. Therefore, there is no requirement for absolute cytochemical specificity of the acceptor fluorophore.

23. J. A. Goodnight, H. Mischak, W. Kolch, J. F. Mushinski (1995) *J Biol Chem* **270,** 9991-10001.

24. T. W. J. Gadella, R. M. Clegg, T. M. Jovin (1994) *Bioimaging* **2,** 139-159.

25. P. Westermann, M. Knoblich, O. Maier, C. Lindschau, H. Haller, (1996) *Biochem J* **320,** 651-658.

26. T. Ng, unpublished observations.

27. P. I. H. Bastiaens, T. M. Jovin (1996) *Proc. Natl. Acad. Sci. USA* **93,** 8407-8412.

28. G. Hansra, F. Bornancin, R. Whelan, B. A. Hemmings, P. J. Parker (1996) *J. Biol. Chem.* **271,** 32785-32788.

29. P. C. Gordge, M. J. Hulme, R. A. Clegg, W. R. Miller (1996) *Eur. J. Cancer* **32A,** 2120-2126.

30. G. Yu *et al* (1991) *Cancer Commun* **3,** 181-9.

31. S. Ahmad, R. I. Glazer (1993) *Mol Pharmacol* **43,** 858-862.

32. G. C. Blobe *et al* (1993) *J. Biol. Chem.* **268,** 658-664.

33. J. Budworth, T. W. Grant, A. Gescher (1997) *Br. J. Cancer* **75,** 1330-1335.

34. To obtain antibodies to the two putative phosphorylation sites, the following synthetic phosphopeptides were synthesised in the Peptide Synthesis Laboratory (ICRF, London): GSLS(P)FGVS-amide, WDRT(P)TRND-amide, where the S(P) and T(P) denote the phosphorylated residues S260 and T250 respectively (see text). Phosphopeptides were coupled to keyhole limpet haemocyanin using glutaraldehyde and the conjugate employed to immunise rabbits. For Western blotting of immobilised proteins Tris-buffered saline pH7.5 was used in place of phosphate-buffered saline. Antibodies were employed at 1/2000 for 1hr at room temperature or overnight at 4°C. Immunoreaction was detected using ECL (Amersham) according to recommended procedures.

35. A. R. Olivier, P. J. Parker (1994) *J Biol Chem* **269,** 2758-2763 .

36. S. C. Kiley, P. D. Adams, P. J. Parker (1997) *Cell Growth and Differentiation* **8,** 221-230.

37. 293 cells were fixed and permeabilised in methanol at -20°C for 4min. Primary antibodies were diluted 1:200 in 10mM Tris-buffered saline containing 1% BSA, except for MC5-Cy3 which was used at 1:50. The secondary conjugates used were Cy2-conjugated donkey anti-mouse IgG (1:200) and Cy3-conjugated donkey anti-rabbit IgG (1:400) (Jackson ImmunoResearch Laboratories, West Grove, PA). Confocal images were acquired on a confocal laser scanning microscope (model LSM410, Carl Zeiss Inc.) equipped with a 63X/1.4Plan-APOCHROMAT oil immersion objective. Each image represents a 2-dimensional projection of sections in the Z-series, taken across the depth of the cell at 0.5mm intervals.

38. P. I. H. Bastiaens, T. M. Jovin in *Cell biology a laboratory handbook* J. E. Celis, Eds. (Academic Press, New York, 1998), pp. 136-146.

39. All fluorescence lifetime images were taken using a Zeiss FLUAR 100X/1.3NA DIC oil objective and the homodyne phase sensitive images recorded at a modulation frequency of 80.218 MHz. For experiments involving the GFP-PKGα/T(P)250-IgG-Cy3.5 donor/acceptor FRET pair, the donor was excited using the 488nm line of the Argon/Krypton laser and the resultant fluorescence separated using a combination of dichroic beamsplitter (Q 505 LP; Chroma Technology Corp.) and narrow band emitter filter (BP514/10; Lys & Optik). Acceptor images were recorded using a 100W Mercury arc lamp (Zeiss Attoarc) as a source of sample illumination combined with a high Q Cy3 filter set (exciter:HQ 535/50, dichroic:Q 565 LP, emitter:HQ 610/75 LP; Chroma Technology Corp.). For FRET experiments involving Cy3/Cy5 donor/acceptor pairs, the 514nm line of the Argon/Krypton laser was used as an excitation source and the resultant fluorescence separated using a combination of dichroic beamsplitter and emitter filter from the high Q Cy3 filter set. For the collection of acceptor images a High Q Cy5 filter set (exciter: HQ 620/60, dichroic:Q 660 LP, emitter:HQ 700/75 LP; Chroma Technology Corp) was used in combination with the mercury lamp. Live cell experiments were performed at 37°C by enclosing the microscope in a temperature stabilised incubator. Microinjections of T(P)250-IgG-Cy3.5 in COS-7 cells were performed *in situ* before FLIM measurements were made by using a combination of an Eppendorf transjector (5246) and micromanipulator (5171).

40. C. Gillett, *et al* (1996) *Int. J. Cancer* **69,** 92-99.

**Example 2: *In situ* detection of ligand-induced activation of the epidermal growth factor receptor (EGF-R or ErbB1)**

A further example of the method is the *in situ* detection of ligand-induced activation of the epidermal growth factor receptor (EGF-R or ErbB1) through its phosphorylation. The ErbB family of receptor tyrosine kinases contains four receptors whose extensive range of receptor-receptor combinations constitutes a signalling network (Alroy, I. And Yarden, Y. (1997) *FEBS Lett.* **410,** 83-86). Upon binding of EGF, ErbB1 dimerizes and transphosphorylates specific tyrosine residues on its cytosolic domain. The phosphotyrosines within the C-terminus of the receptors determine the downstream response by recruiting effector proteins, binding via their Src-homology 2 (SH2) (Pawson, T. (1995) *Nature* **373,** 573-580) and phosphotyrosine-binding (PTB) (Vandergeer, P. and Pawson, T. (1995) *Trends Biochem. Sci.* **20,** 277-280) domains. Another level of signal modulation is endocytosis of receptors after ligand binding (Bevan, P.A. *et al* (1996) *Trends Endocrinol. Metab.* **7,** 13-21).

A C-terminal GFP fusion to the ErbB1 receptor was expressed in NR6 cells lacking the endogenous receptor. Using a Cy3-labelled phosphotyrosine-specific antibody (PY-72-Cy3), phosphorylation and thus activation of the receptor could be detected *in situ* by measuring FRET between GFP-ErbB1 and PY-72-Cy3 by lifetime imaging of the donor GFP on the ErbB1. Figure 8 depicts the fluorescence intensity distributions of GFP-ErbB1 and PY-72-Cy3 together with the FRET efficiency map 10 minutes after administration of EGF. The two cells shown were in different states of processing the ErbB1 receptors. One cell displayed a plasma membrane distribution, whereas the other cell had endocytosed most of the receptors. The cell that internalized the GFP-ErbB1 showed a diminished FRET signal in comparison with the cell with membrane-localized receptors. Since GFP-ErbB1 colocalized with the PY-72-Cy3 fluorescence distribution, the decrease in the FRET signal was probably due to the recruitment of adaptors to phosphorylated tyrosines occluding antibody epitopes on the activated receptors.

## Further Examples

Figure 10 shows that 4F9 (F(ab')$_2$ fragments recognise the activated form of PKC$\alpha$ expressed in MCF-7 cells and that they can be used to monitor the degree of PKC$\alpha$ activation in MCF10A cells by the detection of fluorescence resonance energy transfer (FRET) (see Figure legend for more details).

Ng *et al* (1999) *EMBO J.* **18,** 3909-3923 provides an example of the detection of an interaction between $\beta_1$ integrin and PKC$\alpha$ using the methods of the invention.

**Claims**

1.  A mothod of detecting the state; or the change in state, of a macromolecular component of a cell which can exist in a first state or a second state which differ by virtue of a covalent modification or which are different conformations wherein in its first state the macromolecular component is substantially incapable of being bound by a sensor molecule and in its second state the macromolecular component is able to be bound by the sensor molecule, the method comprising the steps of

    (1) providing a sample comprising the macromolecular component wherein the macromolecular component is bound to a donor fluorophore;
    (2) contacting the sample provided in (1) with a sensor molecule which is able to bind the macromolecular component in its second state but is substantially incapable of binding the macromolecular component in its first state wherein the sensor molecule is bound to an acceptor molecule; and
    (3) detecting fluorescence resonance energy transfer (FRET) between the donor fluorophore bound to the macromolecular component and the acceptor molecule bound to the sensor molecule wherein FRET is measured by the decrease in the fluorescence lifetime of the donor fluorophore.

2.  A method according to Claim 1 wherein the decrease in the fluorescence lifetime of the donor fluorophore is measured using fluorescence lifetime imaging.

3.  A method according to Claim 2 wherein the decrease in the lifetime of the donor fluorophore is measured by fluorescence lifetime imaging microscopy (FLIM).

4.  A method according to Claim 2 or 3 wherein the fluorescence lifetime measurement is spatially resolved.

5.  A method according to any one of Claims 1 to 4 wherein the donor fluorophore exhibits multi-exponential decay and the characteristic lifetime of the decay components are detected using multi-frequency fluorescence lifetime imaging microscopy (mfFLIM).

6.  A method according to any one of Claims 1 to 5 wherein the macromolecular component of a cell is any one of a protein, a RNA molecule, a DNA molecule or a lipid.

7.  A method according to Claim 6 wherein the macromolecular component is a protein which in its first state is not post-translationally modified and in its second state is post-translationally modified.

8. A method according to Claim 7 wherein the post-translational modification is any one of phosphorylation, acetylation, methylation, sulphation, glycosylation, farnesylation, attachment of a fatty acid moiety, and ubiquitinylation.

9. A method according to Claim 6 wherein the macromolecular component of a cell is a lipid which in its first state lacks a given phosphate residue and in its second state possesses the given phosphate residue.

10. A method according to Claim 6 wherein the macromolecular component of a cell is DNA which in its first state is substantially devoid of methylation in a given region and in its second state is methylated in the given region.

11. A method according to Claim 6 wherein the macromolecular component is a protein which in its first state does not have a given epitope accessible to an antibody and its second state has the given epitope accessible to an antibody.

12. A method according to any one of the preceding claims wherein the macromolecular component is covalently bound to the donor fluorophore.

13. A method according to Claim 12 wherein the macromolecular component is fused with a green fluorescent protein (GFP).

14. A method according to any one of Claims 1 to 11 wherein macromolecular component is non covalently bound to an antibody which is bound to the donor fluorophore.

15. A method according to Claim 14 wherein the donor fluorophore is a non-protein fluorophore.

16. A method according to Claim 14 wherein the non-protein donor fluorophore is Cy3.

17. A method according to any one of the preceding claims wherein the sensor molecule is an antibody.

18. A method according to Claim 17 wherein the antibody is covalently bound to the acceptor molecule.

19. A method according to Claim 18 wherein the acceptor molecule is Cy5.

20. A method according to any one of claims 1-11 wherein the donor fluorophore is indirectly bound to the macromolecular component.

21. A method according to any one of Claims 1 to 17 wherein the acceptor molecule is indirectly bound to the sensor.

22. A method for identifying a compound which modulates the interconversion of a macromolecular component from a first state to a second state which differ by virtue of a covalent modification or which are different conformations the method comprising identifying said compound using the method of any one of Claims 1 to 21.

23. A method according to Claim 1 wherein the activation state of protein kinase C$\alpha$ is determined in a sample by detecting the phosphorylation state of threonine residue 250 (T250).

24. A method according to Claim 1 wherein the phosphorylation state of the EGF receptor is determined by using an anti-phosphotyrosine antibody as the sensor molecule.

25. A method according to any one of the preceding claims wherein the sample is a live cell.

26. A kit of parts comprising (a) a macromolecular component covalently linked to a donor fluorophore or a nucleic acid molecule encoding a macromolecular component protein fused to a donor fluorophore protein and (b) a sensor molecule covalently linked to an acceptor molecule or a nucleic acid molecule encoding a sensor protein fused to an acceptor protein, wherein the macromolecular component can exist in a first state or a second state which differ by virtue of a covalent modification or which are different conformations wherein in its first state the macromolecular component is substantially incapable of being bound by the sensor molecule and in its second state the macromolecular component is able to be bound by the sensor molecule.

27. A kit according to Claim 26 wherein the macromolecular component protein is fused to a green fluorescent protein.

**EP 1 145 004 B1**

**28.** A kit according to Claim 26 wherein the sensor molecule is an antibody which can detect phosphorylation of a protein.

**Patentansprüche**

**1.** Verfahren zum Erfassen des Zustandes oder der Veränderung des Zustandes einer makromolekularen zellulären Komponente, die in einem ersten Zustand oder einem zweiten Zustand vorliegen kann, wobei diese Zustände sich auf Grund einer kovalenten Modifikation unterscheiden oder unterschiedliche Konformationen sind, wobei die makromolekulare Komponente in ihrem ersten Zustand im wesentlichen unfähig ist, von einem Sensormolekül gebunden zu werden, und die makromolekulare Komponente in ihrem zweiten Zustand fähig ist, vom Sensormolekül gebunden zu werden, wobei das Verfahren die folgenden Schritte umfasst

(1) Bereitstellen einer Probe, die die makromolekulare Komponente aufweist, wobei die makromolekulare Komponente an ein Donor-Fluorophor gebunden ist;

(2) Inkontaktbringen der in (1) bereit gestellten Probe mit einem Sensormolekül, das fähig ist, die makromolekulare Komponente in ihrem zweiten Zustand zu binden, aber im wesentlichen unfähig ist, die makromolekulare Komponente in ihrem ersten Zustand zu binden, wobei das Sensormotekül an ein Akzeptormolekül gebunden ist; und

(3) Erfassen des Fluoreszenz-Resonanz-Energie-Transfers (FRET) zwischen dem an der makromolekularen Komponente gebundenen Donor-Fluorophor und dem am Sensorrnolekül gebundenen Akzeptormolekül, wobei der FRET anhand der Abnahme der Fluoreszenz-Lebensdauer des Donor-Fluorophors gemessen wird.

**2.** Verfahren nach Anspruch 1, wobei die Abnahme der Fluoreszenz-Lebensdauer des Donor-Fluorophors mittels eines Fluoreszenz-Lebensdauer-Imaging gemessen wird.

**3.** Verfahren nach Anspruch 2, wobei die Abnahme der Fluoreszenz-Lebensdauer des Donor-Fluorophors mittels einer Fluoreszenz-Lebensdauer-Imaging-Mikroskopie (FLIM) gemessen wird.

**4.** Verfahren nach Anspruch 2 oder 3, wobei die Messung der Fluoreszenz-Lebensdauer räumlich aufgelöst ist.

**5.** Verfahren nach einem beliebigen der Ansprüche 1 bis 4, wobei das Donor-Fluorophor einen multiexponentiellen Zerfall zeigt und die charakteristische Lebensdauer der Zerfallskomponenten mittels Multifrequenz-Fluoreszenz-Lebensdauer-Imaging-Mikroskopie (mfFLIM) erfasst wird.

**6.** Verfahren nach einem beliebigen der Ansprüche 1 bis 5, wobei die makromolekulare zelluläre Komponente eine beliebige aus der Gruppe ist, die aus einem Protein, einem RNA-Molekül, einem DNA-Molekül oder einem Lipid besteht.

**7.** Verfahren nach Anspruch 6, wobei die makromolekulare Komponente ein Protein ist, das in seinem ersten Zustand nicht posttranslational modifiziert ist und in seinem zweiten Zustand posttranslational modifiziert ist.

**8.** Verfahren nach Anspruch 7, wobei die posttranslationale Modifikation eine beliebige aus der Gruppe ist, die aus einer Phosphorylierung, Acetylierung, Methylierung, Sulfatierung, Glycosylierung, Farnesylierung, dem Anbringen einer Fettsäuregruppe sowie einer Ubiquitinylierung besteht.

**9.** Verfahren nach Anspruch 6, wobei die makromolekulare zelluläre Komponente ein Lipid ist, dem in seinem ersten Zustand ein vorgegebener Phosphatrest fehlt, und das in seinem zweiten Zustand den vorgegebenen Phosphatrest besitzt.

**10.** Verfahren nach Anspruch 6, wobei die makromolekulare zelluläre Komponente DNA ist, die in ihrem ersten Zustand in einem vorgegebenen Bereich im wesentlichen nicht methyliert ist und in ihrem zweiten Zustand in dem vorgegebenen Bereich methyliert ist.

**11.** Verfahren nach Anspruch 6, wobei die makromolekulare zelluläre Komponente ein Protein ist, das in seinem ersten Zustand kein vorgegebenes Epitop hat, das für einen Antikörper zugänglich ist, und in seinem zweiten Zustand

23

das vorgegebene Epitop hat, das für einen Antikörper zugänglich ist.

12. Verfahren nach einem beliebigen der vorangehenden Ansprüche, wobei die makromolekulare Komponente kovalent an das Donor-Fluorophor gebunden ist

13. Verfahren nach Anspruch 12, wobei die makromolekulare Komponente mit einem grün fluoreszierenden Protein (GFP) fusioniert ist.

14. Verfahren nach einem beliebigen der Ansprüche 1 bis 11, wobei die makromolekulare Komponente nicht-kovalent an einen Antikörper gebunden ist, der an das Donor-Fluorophor gebunden ist.

15. Verfahren nach Anspruch 14, wobei das Donor-Fluorophor ein Fluorophor ist, das kein Protein ist.

16. Verfahren nach Anspruch 14, wobei das Donor-Fluorophor, das kein Protein ist, Cy3 ist.

17. Verfahren nach einem beliebigen der vorangehenden Ansprüche, wobei das Sensormolekül ein Antikörper ist.

18. Verfahren nach Anspruch 17, wobei der Antikörper Kovalent an das Akzeptormolekül gebunden ist.

19. Verfahren nach Anspruch 18, wobei das Akzeptormolekül Cy5 ist.

20. Verfahren nach einem beliebigen der Ansprüche 1 bis 11, wobei das Donor-Fluorophor indirekt an die makromolekulare Komponente gebunden ist.

21. Verfahren nach einem beliebigen der Ansprüche 1 bis 17, wobei das Akzeptormolekül indirekt an den Sensor gebunden ist

22. Verfahren zur Identifizierung einer Verbindung, die die Umwandlung einer makromolekularen Komponente aus einem ersten Zustand in einen zweiten Zustand moduliert, wobei die Zustände sich auf Grund einer kovalenten Modifikation unterscheiden oder unterschiedliche Konformationen sind, wobei das Verfahren das Identifizieren der genannten Verbindung mittels des Verfahrens nach einem beliebigen der Ansprüche 1 bis 21 umfasst.

23. Verfahren nach Anspruch 1, wobei der Aktivierungszustand der Proteinkinase C$_\acute{a}$ in einer Probe über den Nachweis des Phosphorylierungsstatus des Threonin-Restes 250 (T250) bestimmt wird.

24. Verfahren nach Anspruch 1, wobei der Phosphorylierungsstatus des EGF-Rezeptors über die Verwendung eines Anti-Phosphotyrosin-Antikörpers als Sensormolekül bestimmt wird.

25. Verfahren nach einem beliebigen der vorangehenden Ansprüche, wobei die Probe eine lebende Zelle ist.

26. Kit aus Teilen, umfassend (a) eine makromolekulare Komponente, die kovaient mit einem Donor-Fluorophor oder einem Nukleinsäuremolekül verknüpft ist, das für ein Protein als makromolekulare Komponente, das mit einem Donor-Fluorophor-Protein fusioniert ist, codiert, und (b) ein Sensormotekül, das kovalent mit einem Akzeptormolekül oder einem Nukleinsäuremotekül verknüpft ist, das für ein mit einem Akzeptormolekül fusionierten Sensorprotein codiert, wobei die makromolekulare Komponente in einem ersten Zustand oder einem zweiten Zustand, wobei die Zustände sich auf Grund einer kovalenten Modifikation unterscheiden oder unterschiedliche Konformationen sind, vorliegen kann, wobei die makromolekulare Komponente in ihrem ersten Zustand im wesentlichen unfähig ist, vom Sensormotekül gebunden zu werden, und die makromolekulare Komponente in ihrem zweiten Zustand fähig ist, vom Sensormolekül gebunden zu werden.

27. Kit nach Anspruch 26; wobei das Protein als makromolekulare Komponente mit einem grün fluoreszierenden Protein fusioniert ist.

28. Kit nach Anspruch 26, wobei das Sensormolekül ein Antikörper ist, der die Phosphorylierung eines Proteins erfassen kann.

**EP 1 145 004 B1**

**Revendications**

1. Procédé de détection de l'état ou du changement d'état d'un composant macromoléculaire d'une cellule qui peut exister sous un premier état ou sous un second état, qui diffèrent selon une modification covalente ou qui sont sous des conformations différentes, dans lequel, sous son premier état, le composant macromoléculaire est pratiquement incapable d'être lié à une molécule détectrice et sous son second état, le composant macromoléculaire est capable d'être lié à la molécule détectrice, le procédé comprenant les étapes consistant à

   (1) fournir un échantillon comprenant le composant macromoléculaire, dans lequel le composant macromoléculaire est lié à un fluorophore donneur ;
   (2) mettre en contact l'échantillon fourni dans (1) avec une molécule détectrice qui est capable de se lier au composant macromoléculaire sous son second état mais qui est pratiquement incapable de se lier au composant macromoléculaire sous son premier état dans lequel la molécule détectrice est liée à une molécule acceptrice ; et
   (3) détecter le transfert d'énergie de résonance par fluorescence (FRET) entre le fluorophore donneur lié au composant macromoléculaire et la molécule acceptrice liée à la molécule détectrice dans lequel le FRET est mesuré par la diminution de la durée de vie de la fluorescence du fluorophore donneur.

2. Procédé selon la revendication 1, dans lequel la diminution de la durée de vie de la fluorescence du fluorophore donneur est mesurée en utilisant une imagerie de la durée de vie de la fluorescence.

3. Procédé selon la revendication 2, dans lequel la diminution de la durée de vie du fluorophore donneur est mesurée par microscopie par imagerie de la durée de vie de la fluorescence (FLIM).

4. Procédé selon la revendication 2 ou 3, dans lequel la mesure de la durée de vie de la fluorescence est résolue dans l'espace.

5. Procédé selon l'une quelconque des revendications 1 à 4, dans lequel le fluorophore donneur présente une décroissance multi-exponentielle et la durée de vie caractéristique des composants de la décroissance est détectée en utilisant la microscopie par imagerie de la durée de vie de la fluorescence à fréquence multiple (mfFLIM).

6. Procédé selon l'une quelconque des revendications 1 à 5, dans lequel le composant macromoléculaire d'une cellule est choisi parmi une protéine, une molécule d'ARN, une molécule d'ADN ou un lipide.

7. Procédé selon la revendication 6, dans lequel le composant macromoléculaire est une protéine qui sous son premier état n'est pas modifiée après la traduction et sous son second état est modifiée après la traduction.

8. Procédé selon la revendication 7, dans lequel la modification après la traduction est choisi parmi la phosphorylation, l'acétylation, la méthylation, la sulfatation, la glycosylation, la farnésylation, la fixation d'un radical d'acide gras et l'ubiquitinylation.

9. Procédé selon la revendication 6, dans lequel le composant macromoléculaire d'une cellule est un lipide qui sous son premier état est dépourvu d'un résidu phosphate donné et sous son second état possède le résidu phosphate donné.

10. Procédé selon la revendication 6, dans lequel le composant macromoléculaire d'une cellule est l'ADN qui sous son premier état est pratiquement dépourvu de méthylation dans une région donnée et sous son second état est méthylé dans la région donnée.

11. Procédé selon la revendication 6, dans lequel le composant macromoléculaire est une protéine qui sous son premier état n'a pas un épitope donné accessible à un anticorps et sous son second état a l'épitope donné accessible à un anticorps.

12. Procédé selon l'une quelconque des revendications précédentes, dans lequel le composant macromoléculaire est lié de manière covalente au fluorophore donneur.

13. Procédé selon la revendication 12, dans lequel le composant macromoléculaire est fusionné avec une protéine fluorescente verte (GFP).

**14.** Procédé selon l'une quelconque des revendications 1 à 11, dans lequel le composant macromoléculaire est lié de manière non covalente à un anticorps qui est lié au fluorophore donneur.

**15.** Procédé selon la revendication 14, dans lequel le fluorophore donneur est un fluorophore non protéique.

**16.** Procédé selon la revendication 14, dans lequel le fluorophore donneur non protéique est Cy3.

**17.** Procédé selon l'une quelconque des revendications précédentes, dans lequel la molécule détectrice est un anticorps.

**18.** Procédé selon la revendication 17, dans lequel l'anticorps est lié de manière covalente à la molécule acceptrice.

**19.** Procédé selon la revendication 18, dans lequel la molécule acceptrice est Cy5.

**20.** Procédé selon l'une quelconque des revendications 1 à 11, dans lequel le fluorophore donneur est lié indirectement au composant macromoléculaire.

**21.** Procédé selon l'une quelconque des revendications 1 à 17, dans lequel la molécule acceptrice est liée indirectement au détecteur.

**22.** Procédé d'identification d'un composé qui module l'interconversion d'un composant macromoléculaire d'un premier état à un second état qui diffèrent selon une modification covalente ou qui sont sous des conformations différentes, le procédé comprenant l'identification dudit composé en utilisant le procédé selon l'une quelconque des revendications 1 à 21.

**23.** Procédé selon la revendication 1, dans lequel l'état d'activation de la protéine kinase C$\alpha$ est déterminé dans un échantillon en détectant l'état de phosphorylation du résidu thréonine 250 (T250).

**24.** Procédé selon la revendication 1, dans lequel l'état de phosphorylation du récepteur de l'EGF est déterminé en utilisant un anticorps anti-phosphotyrosine comme molécule détectrice.

**25.** Procédé selon l'une quelconque des revendications précédentes, dans lequel l'échantillon est une cellule vivante.

**26.** Trousse d'éléments comprenant (a) un composant macromoléculaire lié de manière covalente à un fluorophore donneur ou une molécule d'acide nucléique codant pour une protéine de composant macromoléculaire fusionnée à une protéine de fluorophore donneur et (b) une molécule détectrice liée de manière covalente à une molécule acceptrice ou une molécule d'acide nucléique codant pour une protéine détectrice fusionnée à une protéine acceptrice, dans laquelle le composant macromoléculaire peut exister sous un premier état ou un second état qui diffèrent selon une modification covalente ou qui sont sous des conformations différentes, dans lequel sous son premier état, le composant macromoléculaire est pratiquement incapable d'être lié à la molécule détectrice et sous son second état, le composant macromoléculaire est capable d'être lié à la molécule détectrice.

**27.** Trousse selon la revendication 26, dans laquelle la protéine de composant macromoléculaire est fusionnée à une protéine fluorescente verte.

**28.** Trousse selon la revendication 26, dans laquelle la molécule détectrice est un anticorps qui peut détecter la phosphorylation d'une protéine.

*Fig. 1A*

TPA (min)  0  2  5  10 20 30 60

→  T(P)250

→  PKCα

# *Fig. 1B*

IP-PKCα
_____

TPA (min)  0  15  30 60 120

→  T(P)250

# *Fig. 1C*

Fig. 1D

TPA (min)  0   1   5   10  30   0

T(P)250

PKCα

*Fig. 2A*

*Fig. 2B*

WT
PKCα

Kinase-
PKCα

*Fig. 3*

Fig. 4A

*Fig. 4B*

*Fig. 5A*

35

*Fig. 5B*

*Fig. 6A*

*Fig. 6B*

*Fig. 7*

*Fig. 8*

State 1

State 2

(a)

(b)

(c)

NO FRET

because no binding
of sensor to
first state

FRET

*Fig. 9*

- TPA              + TPA

GFP-PKCα  4F9(TRITC)  GFP-PKCα  4F9(TRITC)

Acceptor (Cy3)
Photobleaching (min)

MC5-
Oregon Green

⟨τ⟩
(ns)

4F9-Cy3

*Fig. 10*